(19) Europäisches Patentamt
European Patent Office
Office européen des brevets

(11) Veröffentlichungsnummer: **0 172 552**
**A2**

# (12) EUROPÄISCHE PATENTANMELDUNG

(21) Anmeldenummer: **85110370.5**

(22) Anmeldetag: **19.08.85**

(51) Int. Cl.[4]: **C07D 487/04**, C07D 237/04, A61K 31/55, //(C07D487/04,243:00,237:00),(C07D487/04,245:00,237:00),(C07D487/04,255:00,237:00)

(30) Priorität: **24.08.84 GB 8421493**
**29.05.85 GB 8513541**

(43) Veröffentlichungstag der Anmeldung:
**26.02.86 Patentblatt 86/09**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Hassall, Cedric Herbert**
**6 Ashcroft Close**
**Harpenden Herts.(GB)**
Erfinder: **Lawton, Geoffrey**
**109 Whitehill Road**
**Hitchin Herts.(GB)**
Erfinder: **Redshaw, Sally**
**29 Hertford Road**
**Stevenage Herts.(GB)**

(74) Vertreter: **Lederer, Franz, Dr. et al**
**Patentanwälte Dr. Franz Lederer Dipl.-Ing. Reiner F. Meyer-Roxlau Lucile-Grahn-Strasse 22**
**D-8000 München 80(DE)**

## (54) Bicyclische Carbonsäuren und deren Alkylester, Verfahren und Zwischenprodukte zu deren Herstellung sowie diese enthaltende Arzneimittel.

(57) Verbindungen der Formel

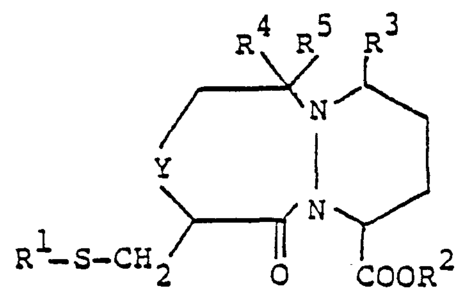

worin $R^1$ Wasserstoff, Alkanoyl oder Aroyl, $R^2$ Wasserstoff oder Alkyl, $R^3$ Wasserstoff oder Aryl, $R^4$ und $R^5$ je Wasserstoff oder zusammen eine Oxogruppe und Y $-CH_2-$, $-CH_2CH_2-$ oder $-N(R^6)-$ bedeuten, worin $R^6$ Wasserstoff, Alkyl oder Aralkyl bedeutet, sowie pharmazeutisch verwendbare Salze davon mit Basen, falls $R^2$ Wasserstoff bedeutet, und pharmazeutisch verwendbare Salze davon mit Säuren, falls Y $-N(R^6)-$ bedeutet, besitzen antihypertensive Eigenschaften und können demnach in Form pharmazeutischer Präparate verwendet werden. Sie können nach bekannten Methoden hergestellt werden.



Rank Xerox

Bicyclische Carbonsäuren und deren Alkylester, Verfahren und Zwischenprodukte zu deren Herstellung sowie diese enthaltende Arzneimittel

Die vorliegende Erfindung betrifft bicyclische Verbindungen, ein Verfahren zu deren Herstellung sowie Arzneimittel enthaltend diese Verbindungen.

Im speziellen betrifft die Erfindung bicyclische Verbindungen der allgemeinen Formel

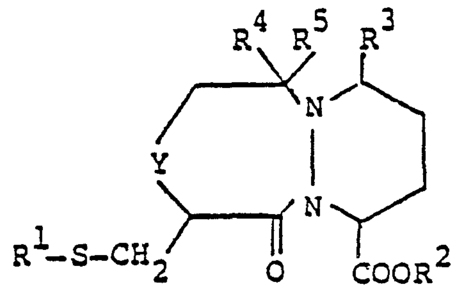

worin $R^1$ Wasserstoff, Alkanoyl oder Aroyl, $R^2$ Wasserstoff oder Alkyl, $R^3$ Wasserstoff oder Aryl, $R^4$ und $R^5$ je Wasserstoff oder zusammen eine Oxogruppe und Y $-CH_2-$, $-CH_2CH_2-$ oder $-N(R^6)-$ bedeuten, worin $R^6$ Wasserstoff, Alkyl oder Aralkyl bedeutet, sowie pharmazeutisch verwendbare Salze davon mit Basen, falls $R^2$ Wasserstoff bedeutet, und pharmazeutisch verwendbare Salze davon mit Säuren, falls Y $-N(R^6)-$ bedeutet.

Die Verbindungen der Formel I enthalten mindestens ein asymmetrisches Kohlenstoffatom. Die vorliegende Erfindung umfasst nicht nur die optisch einheitlichen Formen dieser Verbindungen, sondern auch die verschiedenen diastereoisomeren Racemate und Mischungen der verschiedenen diastereoisomeren Racemate. Die erfindungsgemässen Verbindungen weisen an den asymmetrischen Kohlenstoffatomen, welche die Substituenten $R^1-S-CH_2-$ und $R^2OOC-$ tragen, vorzugsweise die S-Konfiguration auf.

Der in der vorliegenden Beschreibung verwendete Ausdruck "Alkyl" betrifft geradkettige oder verzweigte Alkylreste mit 1-8, vorzugsweise 1-4, Kohlenstoffatomen, wie Methyl, Aethyl, Propyl, Isopropyl, Butyl, tert.Butyl, Pentyl, Hexyl und dergleichen. Der Ausdruck "Aryl" betrifft eine Phenylgruppe, welche durch Halogen, Alkyl, Alkoxy, Trifluormethyl und dergleichen ein- oder mehrfach substituiert sein kann. Beispiele von Arylgruppen sind Phenyl, 4-Chlorphenyl, p-Tolyl, 4-Methoxyphenyl und dergleichen. Der Ausdruck "Aralkyl" betrifft Alkylgruppen, in denen eines der Wasserstoffatome durch eine Arylgruppe ersetzt ist. Beispiele von Aralkylgruppen sind Benzyl, 2-Phenyläthyl, 3-Phenylpropyl, 4-Chlorbenzyl, 4-Methoxybenzyl und dergleichen. Der Ausdruck "Alkanoyl" betrifft den Acylrest einer Alkancarbonsäure mit bis zu 8, vorzugsweise 4, Kohlenstoffatomen, wie Essigsäure, Propionsäure, Buttersäure, Valeriansäure, Pivalinsäure, etc. Der Ausdruck "Aroyl" betrifft den Acylrest von Benzoesäure, welche durch Halogen, Alkyl, Alkoxy, Trifluormethyl und dergleichen ein- oder mehrfach substituiert sein kann, wie 4-Chlorbenzoesäure, p-Toluylsäure, 4-Methoxybenzoesäure, etc. Der Ausdruck "Alkoxy" betrifft Alkylgruppen, welche über ein Sauerstoffatom gebunden sind, wie Methoxy, Aethoxy, Propoxy, Isopropoxy, Butoxy, tert.Butoxy, Pentyloxy, Hexyloxy, etc. Der Ausdruck "Halogen" betrifft die vier Halogene Fluor, Chlor, Brom und Jod.

Die Verbindungen der Formel I, worin $R^2$ Wasserstoff bedeutet, bilden pharmazeutisch verwendbare Salze mit Basen. Beispiele solcher Salze sind Alkalimetallsalze, z.B. Natrium- und Kaliumsalze, Erdalkalimetallsalze, z.B. Calcium- und Magnesiumsalze, Ammoniumsalze und Salze mit organischen Basen, wie Dicyclohexylamin etc. Die Verbindungen der Formel I, worin Y $-N(R^6)-$ bedeutet, bilden pharmazeutisch verwendbare Salze mit Säuren, wie Mineralsäuren, z.B. Salzsäure, Bromwasserstoffsäure, Schwefelsäure, Salpetersäure und Phosphorsäure, und organischen Säuren, z.B. Essigsäure, Maleinsäure, Fumarsäure, Bernsteinsäure, Weinsäure, Zitronensäure, Methansulfonsäure, p-Toluolsulfonsäure, etc.

Eine bevorzugte Klasse von erfindungsgemässen Verbindungen sind solche, worin $R^1$ Wasserstoff bedeutet. $R^2$ bedeutet vorzugsweise Wasserstoff. Was $R^3$ anbelangt, so bedeutet dieses bevorzugt Wasserstoff. Y bedeutet vorzugsweise $-CH_2-$.

Aus dem obigen folgt, dass besonders bevorzugte Verbindungen der Formel I solche sind, worin $R^1$, $R^2$ und $R^3$ je Wasserstoff und Y $-CH_2-$ bedeuten.

Ganz besonders bevorzugte Verbindungen der Formel I sind:

Octahydro-9-mercaptomethyl-6,10-dioxo-6H-pyridazo[-1,2-a][1,2]diazepin-1-carbonsäure und

Octahydro-9-mercaptomethyl-10-oxo-6H-pyridazo[1,2--a][1,2]diazepin-1-carbonsäure.

Weitere interessante Verbindungen der Formel I sind:

9-Acetylthiomethyl-octahydro-6,10-dioxo-6H-pyridazo[-1,2-a] [1,2]diazepin-1-carbonsäure,

9-Acetylthiomethyl-octahydro-10-oxo-6H-pyridazo[1,2--a][1,2]diazepin-1-carbonsäure,

tert.Butyl 9-acetylthiomethyl-octahydro-6,10-dioxo-6H-pyridazo[1,2-a]-[1,2]diazepin-1-carboxylat und

tert.Butyl 9-acetylthiomethyl-octahydro-10-oxo-6H-pyridazo[1,2-a][1,-2]diazepin-1-carboxylat.

Weitere Beispiele interessanter Verbindungen der Formel I sind:

Decahydro-10-mercaptomethyl-6,11-dioxo-6H-pyridazo-[1,2-a][1,2]diazocin-1-carbonsäure,

tert.Butyl 10-acetylthiomethyl-decahydro-6,11-dioxo-6H-pyridazo[1,2--a][1,2]diazocin-1-carboxylat,

9-Acetylthiomethyl-8-benzyl-octahydro-6,10-dioxo-6H--pyridazo[1,2-a][1,2,5]triazepin-1-carbonsäure,

8-Benzyl-octahydro-9-mercaptomethyl-6,10-dioxo-6H--pyridazo[1,2-a][1,2,5]triazepin-1-carbonsäure,

9-Accetylthiomethyl-8-methyl-octahydro-6,10-dioxo-6H--pyridazo[1,2-a][1,2,5]triazepin-1-carbonsäure,

Octahydro-9-mercaptomethyl-8-methyl-6,10-dioxo-6H--pyridazo[1,2-a][1,2,5]triazepin-1-carbonsäure,

tert.Butyl 9-acetylthiomethyl-8-benzyl-octahydro-6,10-dioxo-6H-pyridazo[1,2-a][1,2,5]triazepin-1-carboxylat,

tert.Butyl 9-acetylthiomethyl-8-methyl-octahydro-6,10-dioxo-6H-pyridazo[1,2-a][1,2,5]triazepin-1-carboxylat und

Methyl 9-acetylthiomethyl-octahydro-6,10-dioxo-6H-pyridazo[1,2,a]-[1,2]diazepin-1-carboxylat.

Die Verbindungen der Formel I und deren pharmazeutisch verwendbare Salze können erfindungsgemäss dadurch hergestellt werden, dass man

a) zur Herstellung einer Verbindung der Formel I, worin $R^4$ und $R^5$ zusammen eine Oxogruppe bedeuten, eine Verbindung der allgemeinen Formel

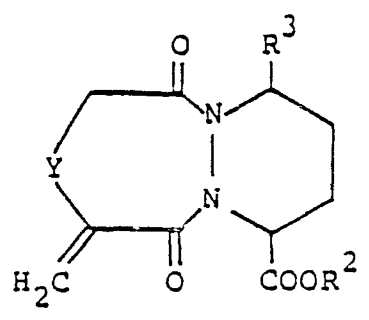

worin $R^2$, $R^3$ und Y die oben angegebene Bedeutung besitzen, mit einer Verbindung der allgemeinen Formel

$R^1$-SH

III

worin $R^1$ die oben angegebene Bedeutung besitzt, umsetzt, oder

b) zur Herstellung einer Verbindung der Formel I, worin $R^1$ Alkanoyl oder Aroyl, $R^2$ Alkyl und Y $-CH_2-$, $-CH_2CH_2-$ oder $-N(R^6)-$ bedeuten, worin $R^6$ Alkyl oder Aralkyl bedeutet, eine Verbindung der allgemeinen Formel

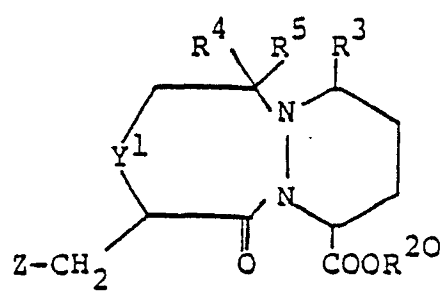

worin $R^3$, $R^4$ und $R^5$ die oben angegebene Bedeutung besitzen, $R^{20}$ Alkyl, $Y^1$ $-CH_2-$, $-CH_2CH_2-$ oder $-N(R^{60})-$, worin $R^{60}$ Alkyl oder Aralkyl bedeutet, und Z eine Abgangsgruppe bedeuten, mit einem Alkalimetallsalz einer Verbindung der allgemeinen Formel

$R^{10}$-SH

V

worin $R^{10}$ Alkanoyl oder Aroyl bedeutet, umsetzt, oder

c) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

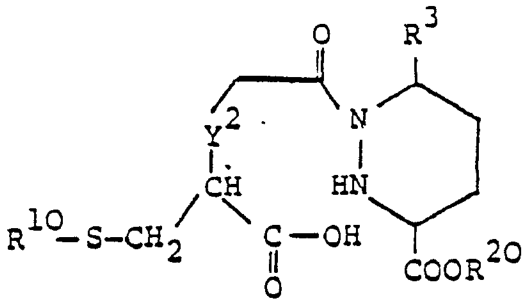

worin $R^3$, $R^{10}$ und $R^{20}$ und die oben angegebene Bedeutung besitzen und $Y^2$ $-CH_2-$ oder $-CH_2CH_2-$ bedeutet, cyclisiert, oder

d) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

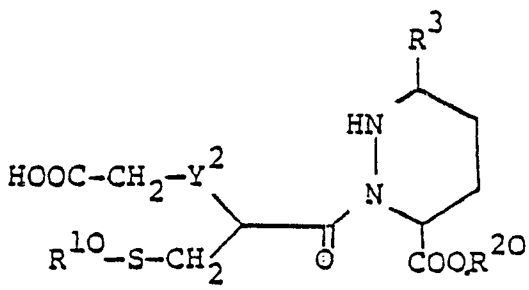

worin $R^3$, $R^{10}$, $R^{20}$ und $Y^2$ die oben angegebene Bedeutung besitzen, cyclisiert, oder

e) zur Herstellung einer Verbindung der Formel I, worin $R^1$ Alkanoyl oder Aroyl, $R^2$ Alkyl, $R^4$ und $R^5$ zusammen eine Oxogruppe und Y -NH- bedeuten, eine Verbindung der allgemeinen Formel

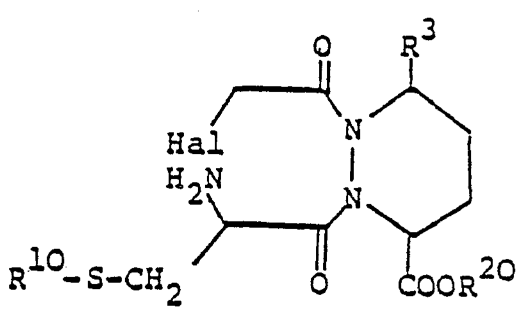

worin $R^3$, $R^{10}$ und $R^{20}$ die oben angegebene Bedeutung besitzen und Hal Halogen bedeutet, cyclisiert, oder

f) zur Herstellung einer Verbindung der Formel I, worin $R^1$ Alkanoyl oder Aroyl, $R^2$ Alkyl und $R^4$ und $R^5$ je Wasserstoff bedeuten, eine Verbindung der Formel I, worin $R^1$ Alkanoyl oder Aroyl, $R^2$ Alkyl und $R^4$ und $R^5$ zusammen eine Oxogruppe bedeuten, reduziert, oder

g) zur Herstellung einer Verbindung der Formel I, worin $R^1$ Alkanoyl oder Aroyl und Y $-N(R^6)-$ bedeuten, worin $R^6$ Alkyl oder Aralkyl bedeutet, eine Verbindung der Formel I, worin $R^1$ Alkanoyl oder Aroyl und Y -NH- bedeuten, mit einer Verbindung der allgemeinen Formel

$R^{60}$-Z IX

worin $R^{60}$ und Z die oben angegebene Bedeutung besitzen, umsetzt, oder

h) zur Herstellung einer Verbindung der Formel I, worin $R^1$ Wasserstoff bedeutet, eine Verbindung der Formel I, worin $R^1$ Alkanoyl oder Aroyl bedeutet, mit einer Base behandelt, oder

i) zur Herstellung einer Verbindung der Formel I, worin $R^2$ Wasserstoff bedeutet, eine Verbindung der Formel I, worin $R^2$ Alkyl bedeutet, mit einer Säure oder einer Base behandelt, oder

j) zur Herstellung einer Verbindung der Formel I, worin $R^1$ Alkanoyl oder Aroyl und Y $-CH_2-$, $-CH_2CH_2-$ oder $-N(R^6)-$ bedeuten, worin $R^6$ Alkyl oder Aralkyl bedeutet, eine entsprechende Verbindung der Formel I, worin $R^1$ Wasserstoff bedeutet, alkanoyliert oder aroyliert, oder

k) erwünschtenfalls ein erhaltenes Gemisch von diastereoisomeren Racematen in die entsprechenden diastereoisomeren Racemate oder optisch reinen Diastereoisomeren auftrennt, und/oder

l) erwünschtenfalls ein erhaltenes Racemat in die beiden optischen Antipoden auftrennt, und

m) erwünschtenfalls eine erhaltene Verbindung der Formel I, worin $R^2$ Wasserstoff bedeutet, in ein pharmazeutisch verwendbares Salz mit einer Base überführt oder eine erhaltene Verbindung der Formel I, worin Y $-N(R^6)-$ bedeutet, in ein pharmazeutisch verwendbares Salz mit einer Säure überführt.

Die Umsetzung einer Verbindung der Formel II mit einer Verbindung der Formel III gemäss Verfahrensvariante a) erfolgt in an sich bekannter Weise. Die Umsetzung kann in Gegenwart oder Abwesenheit eines inerten organischen Lösungsmittels durchgeführt werden. Falls ein inertes orga nisches Lösungsmittel verwendet wird, so kommen beispielsweise ein aliphatischer oder aromatischer Kohlenwasserstoff, z.B. n-Hexan, Benzol, Toluol, etc., ein halogenierter Kohlenwasserstoff, z.B. Methylenchlorid, Chloroform, 1,1,1-Trichloräthan, etc., ein aliphatischer oder cyclischer Aether, z.B. Diäthyläther, Glykoldimethyläther, Tetrahydrofuran, Dioxan, etc., ein Amid, z.B. Dimethylformamid, ein aliphatisches Keton, z.B. Aceton, Methyläthylketon, etc., und dergleichen in Frage. Bevorzugte Lösungsmittel sind halogenierte Kohlenwasserstoffe, insbesondere Methylenchlorid. Die Reaktionstemperatur ist nicht kritisch und kann zwischen etwa Raumtemperatur und der Rückflusstemperatur des Reaktionsgemisches liegen. In einer bevorzugten Ausführungsform wird die Reaktion bei ungefähr Raumtemperatur durchgeführt.

Als Abgangsgruppe Z in den in Verfahrensvariante b) als Ausgangsstoffe eingesetzten Verbindungen der Formel IV kommt beispielsweise ein Halogenatom, wie Chlor oder Brom, eine Alkylsulfonatgruppe, wie Methansulfonyloxy, oder eine Arylsulfonatgruppe, wie p-Toluolsulfonyloxy, in Frage.

Die Umsetzung einer Verbindung der Formel IV mit einem Alkalimetallsalz einer Verbindung der Formel V gemäss Verfahrensvariante b) wird zweckmässigerweise in Gegenwart eines inerten organischen Lösungsmittels durchgeführt. Das Alkalimetallsalz kann beispielsweise das Natriumsalz oder, vorzugsweise, das Kaliumsalz, sein. Die Umsetzung kann zweckmässigerweise in Gegenwart einer katalytischen Menge eines Alkalimetalljodids, z.B. Natriumjodid, Kaliumjodid etc., durchgeführt werden. Geeignete inerte organische Lösungsmittel sind aliphatische Ketone, z.B. Aceton, Methyläthylketon etc., halogenierte Kohlenwasserstoffe, z.B. Methylenchlorid, Chloroform, 1,1,1-Trichloräthan etc., aliphatische Ester, z.B. Aethylacetat, Butylacetat etc., Amide, z.B. Dimethylformamid etc., und dergleichen. Die Reaktionstemperatur ist nicht kritisch. Die Reaktion kann beispielsweise bei einer Temperatur zwischen etwa 10°C und der Rück flusstemperatur des Reaktionsgemisches durchgeführt werden. In einer bevorzugten Ausführungsform wird die Reaktion bei der Rückflusstemperatur des Reaktionsgemisches durchgeführt.

Die Cyclisation einer Verbindung der Formel VI gemäss Verfahrensvariante c) erfolgt ebenfalls nach bekannten Methoden. Gemäss einer bevorzugten Ausführungsform wird die Cyclisation so durchgeführt, dass man eine Verbindung der Formel VI durch Umsetzen in bekannter Weise mit einem geeigneten Halogenierungsmittel, wie einem Phosphorpentahalogenid, z.B. Phosphorpentachlorid etc., oder einem Thionylhalogenid, z.B. Thionylchlorid etc., in das entsprechende Säurehalogenid, z.B. Säurechlorid, überführt, welches spontan zur erwünschten Verbindung der Formel I cyclisiert.

Die Cyclisation einer Verbindung der Formel VII gemäss Verfahrensvariante d) erfolgt ebenfalls in an sich bekannter Weise. In einer bevorzugten Ausführungsform wird die Cyclisation so durchgeführt, dass man eine Verbindung der Formel VII durch Umsetzen in bekannter Weise mit einem geeigneten Halogenierungsmittel, wie einem Phosphorpentahalogenid, z.B. Phosphorpentachlorid etc., oder einem Thionylhalogenid, z.B. Thionylchlorid etc., in das entsprechende Säurehalogenid, z.B. Säurechlorid, überführt, welches dann spontan zur erwünschten Verbindung der Formel I cyclisiert.

Die Cyclisation einer Verbindung der Formel VIII gemäss Verfahrensvariante e) erfolgt leicht, beispielsweise in Gegenwart einer Base, wie eines wässrigen Alkalimetallcarbonats oder -bicarbonats, z.B. Natriumbicarbonat etc., und in Gegenwart eines inerten organischen Lösungsmittels, wie einem aliphatischen oder aromatischen Kohlenwasserstoff, z.B. n-Hexan, Benzol, Toluol etc., einem halogenierten Kohlenwasserstoff, z.B. Methylenchlorid. Chloroform, 1,1,1-Trichloräthan etc., einem aliphatischen oder cyclischen Aether, z.B. Diäthyläther, Glykoldimethyläther, Tetrahydrofuran, Dioxan etc., einem Amid, z.B. Dimethylformamid, einem aliphatischen Keton, z.B. Aceton, Methyläthylketon etc., oder dergleichen. Obwohl die Cyclisation vorzugsweise bei ungefähr Raumtemperatur durchgeführt wird, kann sie erwünschtenfalls auch bei erhöhter Temperatur, beispielsweise bei einer Temperatur bis zur Rückflusstemperatur des Reaktionsgemisches, durchgeführt werden.

Die Reduktion einer Verbindung der Formel I, worin $R^1$ Alkanoyl oder Aroyl, $R^2$ Alkyl und $R^4$ und $R^5$ zusammen eine Oxogruppe bedeuten, gemäss Verfahrensvariante f) erfolgt zweckmässig unter Verwendung von Boran, vorzugsweise einem Borankomplex, wie Boran/Tetrahydrofuran, Boran/Dimethylsulfid, Boran/N,N-Diäthylanilin oder einem ähnlichen Komplex. Diese Reduktion mit Boran wird vorzugsweise in einem inerten organischen Lösungsmittel bei tiefer Temperatur durchgeführt, beispielsweise unter Verwendung eines Boran/Tetrahydrofuran-Komplexes in Tetrahydrofuran bei ungefähr 0°C bis 20°C.

Die Umsetzung einer Verbindung der Formel I, worin $R^1$ Alkanoyl oder Aroyl und Y -NH- bedeuten, mit einer Verbindung der Formel IX gemäss Verfahrensvariante g) erfolgt nach bekannten Methoden. Die Reaktion wird zweckmässig in Gegenwart eines inerten organischen Lösungsmittels durchgeführt. Beispiele solcher Lösungsmittel sind ein halogenierter Kohlenwasserstoff, z.B. Methylenchlorid, Chloroform etc., ein Amid, z.B. Dimethylformamid etc., ein Nitril, z.B. Acetonitril etc., oder dergleichen. Erwünschtenfalls kann die Reaktion in Gegenwart von einer organischen Base, wie einem Trialkylamin, z.B. Triäthylamin, Diäthylisopropylamin etc., Pyridin, N,N-Dimethylanilin etc., durchgeführt werden. Die Reaktion kann bei ungefähr Raumtemperatur oder einer erhöhten Temperatur, beispielsweise der Rückflusstemperatur des Reaktionsgemisches, erfolgen.

Die Ueberführung einer Verbindung der Formel I, worin $R^1$ Alkanoyl oder Aroyl bedeutet, in eine Verbindung der Formel I, worin $R^1$ Wasserstoff bedeutet, gemäss Verfahrensvariante h) erfolgt durch Umsetzen mit einer Base. Diese Umsetzung erfolgt ebenfalls in an sich bekannter Weise. Geeignete Basen sind Alkalimetallhydroxide, z.B.

Natriumhydroxid und Kaliumhydroxid, sowie Ammoniumhydroxid. Die Umsetzung kann bei einer Temperatur zwischen ungefähr Raumtemperatur und dem Siedepunkt des Reaktionsgemisches, vorzugsweise bei der Raumtemperatur, erfolgen.

Die Ueberführung einer Verbindung der Formel I, worin $R^2$ Alkyl bedeutet, in eine Verbindung der Formel I, worin $R^2$ Wasserstoff bedeutet, gemäss Verfahrensvariante i) erfolgt ebenfalls in an sich bekannter Weise durch Umsetzen mit einer Base oder, falls die Alkylgruppe tert.Butyl ist, durch Umsetzen mit einer Säure. Diese Umsetzung erfolgt ebenfalls nach bekannten Methoden. Geeignete Basen sind Alkalimetallhydroxide, z.B. Natriumhydroxid und Kaliumhydroxid, sowie Ammoniumhydroxid. Die Umsetzung erfolgt bei einer Temperatur zwischen ungefähr Raumtemperatur und dem Siedepunkt des Reaktionsgemisches, zweckmässigerweise bei ungefähr Raumtemperatur. Wasserfreie Trifluoressigsäure ist eine speziell geeignete Säure für diesen Zweck, wobei in dem Fall die Umsetzung zweckmässigerweise bei ungefähr Raumtemperatur durchgeführt wird.

Für die Alkanoylierung oder Aroylierung einer Verbindung der Formel I, worin $R^1$ Wasserstoff bedeutet, gemäss Verfahrensvariante j) kommen bekannte Methoden in Frage. Beispielsweise kann die Alkanoylierung so durchgeführt werden, dass man eine Verbindung der Formel I mit einem reaktiven Derivat einer geeigneten Säure, z.B. dem Säurechlorid, in Gegenwart einer Base in einem inerten organischen Lösungsmittel umsetzt. Geeignete Basen sind beispielsweise organische Basen, wie Trialkylamine, z.B. Triäthylamin etc., Pyridin, N,N-Dimethylanilin etc. Geeignete inerte organische Lösungsmittel sind beispielsweise halogenierte Kohlenwasserstoffe, z.B. Methylenchlorid, Chloroform etc., Amide, z.B. Dimethylformamid etc., und dergleichen. Die Temperatur, bei der diese Reaktion durchgeführt wird, ist nicht kritisch und kann zwischen ungefähr Raumtemperatur und der Rückflusstemperatur des Reaktionsgemisches variieren.

Die Auftrennung von diastereoisomeren Mischungen in die diastereoisomeren Racemate oder optisch reinen Diastereoisomeren gemäss Verfahrensvariante k) erfolgt nach an sich bekannten Methoden, beispielsweise durch Chromatographie, z.B. an Silicagel, unter Verwendung eines geeigneten Lösungsmittelsystems, z.B. Aethylacetat/n-Hexan.

Die Auftrennung eines Racemates in die beiden optischen Antipoden gemäss Verfahrensvariante 1) kann in bekannter Weise durchgeführt werden, beispielsweise durch Umsetzen mit einer geeigneten optisch aktiven Säure oder einer geeigneten optisch aktiven Base in Abhängigkeit von der Natur des zu spaltenden Racemates, Abtrennen des erhaltenen optisch aktiven Salzes, z.B. durch fraktionierte Kristallisation, und nötigenfalls Freisetzen der optisch einheitlichen Verbindung aus diesem Salz nach bekannten Methoden.

Die Ueberführung einer Verbindung der Formel I, worin $R^2$ Wasserstoff bedeutet, in ein pharmazeutisch verwendbares Salz mit einer Base und die Ueberführung einer Verbindung der Formel I, worin Y -N($R^6$)- bedeutet, in ein pharmazeutisch verwendbares Salz mit einer Säure gemäss Verfahrensvariante m) erfolgt durch Umsetzen mit einer geeigneten Base oder Säure in bekannter Weise.

Die in Verfahrensvariante a) als Ausgangsstoffe eingesetzten Verbindungen der Formel II sind neu und ebenfalls Gegenstand der vorliegenden Erfindung.

Die Verbindungen der Formel II können hergestellt werden, indem man beispielsweise die Benzyloxycarbonylgruppe aus einer Verbindung der allgemeinen Formel

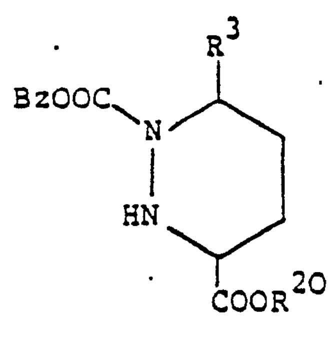

worin $R^3$ und $R^{20}$ die oben angegebene Bedeutung besitzen und Bz Benzyl bedeutet, abspaltet, die erhaltene Verbindung der allgemeinen Formel

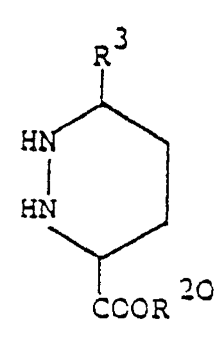

worin $R^3$ und $R^{20}$ die oben angegebene Bedeutung besitzen, mit einem Anhydrid der allgemeinen Formel

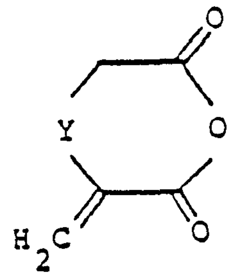

worin Y die oben angegebene Bedeutung besitzt, umsetzt, die dabei erhaltene Verbindung der allgemeinen Formel

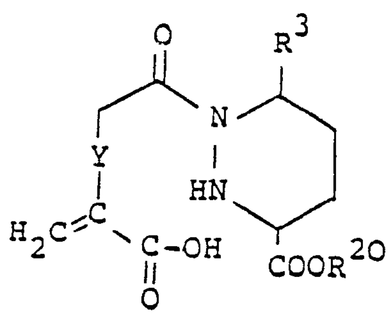

worin $R^3$, $R^{20}$ und Y die oben angegebene Bedeutung besitzen, cyclisiert und, erwünschtenfalls, die so erhaltene Verbindung der Formel II, worin $R^2$ Alkyl bedeutet, mit einer Säure oder einer Base behandelt, wobei eine entsprechende Verbindung der Formel II, worin $R^2$ Wasserstoff bedeutet, erhalten wird und diese Verbindung, erwünschtenfals, durch Veresterung in eine entsprechende Verbindung der Formel II überführt, worin $R^2$ Alkyl bedeutet.

Die Abspaltung der Benzyloxycarbonylgruppe aus einer Verbindung der Formel X erfolgt nach bekannten Methoden, beispielsweise mittels Wasserstoff in Gegenwart eines Katalysators, wie eines Edelmetallkatalysators, z.B. Palladium auf Kohle, und in Gegenwart eines inerten organischen Lösungsmittels, z.B. eines Alkanols, wie Methanol etc.

Die erhaltene Verbindung der Formel XI wird dann mit einem Anhydrid der Formel XII zu einer Verbindung der Formel XIII umgesetzt. Diese Umsetzung erfolgt zweckmässigerweise in einem inerten organischen Lösungsmittel, wie einem halogenierten Kohlenwasserstoff oder, vorzugsweise, einem aliphatischen oder cyclischen Aether, z.B. Dioxan, bei einer Temperatur zwischen ungefähr Raumtemperatur und der Rückflusstemperatur des Reaktionsgemisches, vorzugsweise bei Raumtemperatur.

Die Cyclisation einer Verbindung der Formel XIII wird nach bekannten Methoden durchgeführt. In einer bevorzugten Ausführungsform erfolgt die Cyclisation, indem man eine Verbindung der Formel XIII durch Umsetzen in bekannter Weise mit einem geeigneten Halogenierungsmittel, wie einem Phosphorpentahalogenid, z.B. Phosphorpentachlorid etc., oder einem Thionylhalogenid, z.B. Thionylchlorid etc., in das entsprechende Säurehalogenid, z.B. Säurechlorid, überführt, welches dann spontan zu einer Verbindung der Formel II cyclisiert, worin $R^2$ Alkyl bedeutet.

Die so erhaltene Verbindung der Formel II, worin $R^2$ Alkyl bedeutet, kann erwünschtenfalls in eine Verbindung der Formel II, worin $R^2$ Wasserstoff bedeutet, übergeführt werden, indem man diese mit einer Säure oder einer Base behandelt. Diese Behandlung erfolgt nach bekannten Methoden. Geeignete Basen sind Alkalimetallhydroxide, z.B. Natriumhydroxid und Kaliumhydroxid, sowie Ammoniumhydroxid. Die Behandlung erfolgt bei einer Temperatur zwischen ungefähr Raumtemperatur und dem Siedepunkt des Reaktionsgemisches, vorzugsweise bei ungefähr Raumtemperatur.

Die Veresterung einer Verbindung der Formel II, worin $R^2$ Wasserstoff bedeutet, erfolgt ebenfalls nach bekannten Methoden, beispielsweise durch Umsetzen mit einem geeigneten Diazoalkan, wie Diazomethan.

Gemäss einem Alternativverfahren zur Herstellung von Verbindungen der obigen Formel XIII, worin $R^{20}$ tert.Butyl und Y $-CH_2-$ oder $-CH_2CH_2-$ bedeuten, wird eine Verbindung der obigen Formel XI, worin $R^{20}$ tert.Butyl bedeutet, mit einem $C_{1-3}$-Alkyl-Halbester einer 2-Methylen-pentan- oder -hexandicarbonsäure umgesetzt und das erhaltene Produkt mit einer Base behandelt.

Diese Umsetzung einer Verbindung der Formel XI, worin $R^{20}$ tert.Butyl bedeutet, mit einem Halbester erfolgt in bekannter Weise. Zweckmässigerweise wird der oben genannte Halbester zunächst in ein reaktives Derivat, wie einen aktivierten Ester, übergeführt, welches danach mit der Verbindung der Formel XI umgesetzt wird. Diese Umsetzung erfolgt zweckmässigerweise in einem inerten organischen Lösungsmittel, z.B. einem Aether, wie Tetrahydrofuran etc., und bei einer Temperatur zwischen ungefähr 0°C und der Raumtemperatur.

Die Umsetzung des Reaktionsprodukts mit einer Base unter Bildung einer Verbindung der Formel XIII, worin $R^{20}$ tert.Butyl und Y $-CH_2-$ oder $-CH_2CH_2-$ bedeuten, kann in analoger Weise erfolgen, wie dies weiter oben im Zusammenhang mit der Ueberführung einer Verbindung der Formel II, worin $R^2$ Alkyl bedeutet, in eine Verbindung der Formel II, worin $R^2$ Wasserstoff bedeutet, mittels Basenbehandlung beschrieben worden ist.

Die in Verfahrensvariante b) als Ausgangsstoffe verwendeten Verbindungen der Formel IV sind neu und ebenfalls Gegenstand der vorliegenden Erfindung.

Die Verbindungen der Formel IV, worin Y $-CH_2-$ oder $-CH_2CH_2-$ bedeutet, können beispielsweise hergestellt werden, indem man eine Verbindung der obigen Formel X mit einer Verbindung der allgemeinen Formel

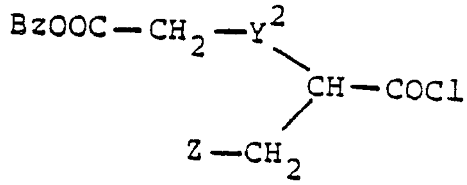

worin $Y^2$, Z und Bz die oben angegebene Bedeutung besitzen, umsetzt, die Benzyl- und Benzyloxycarbonylgruppen aus der erhaltenen Verbindung der allgemeinen Formel

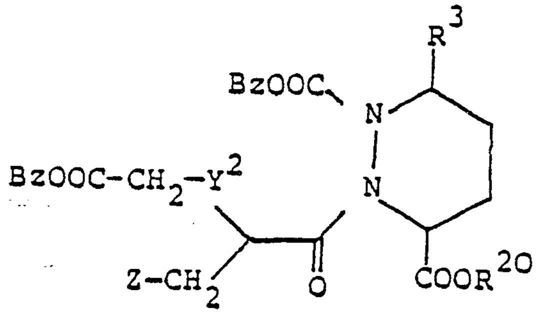

worin $R^3$, $R^{20}$, $Y^2$, Z und Bz die oben angegebene Bedeutung besitzen, abspaltet und die erhaltene Säure der allgemeinen Formel

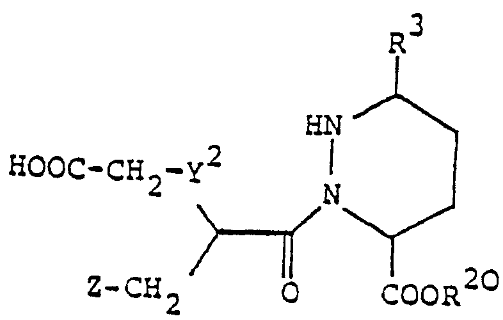

worin $R^3$. $R^{20}$, $Y^2$ und Z die oben angegebene Bedeutung besitzen, cyclisiert und, erwünschtenfalls, die erhaltene Verbindung der Formel IV, worin $R^4$ und $R^5$ zusammen eine Oxogruppe und $Y^1$ $-CH_2-$ oder $-CH_2CH_2-$ bedeuten, zu einer entsprechendn Verbindung der Formel IV, worin $R^4$ und $R^5$ je Wasserstoff bedeuten, reduziert.

Die Umsetzung einer Verbindung der Formel X mit einer Verbindung der Formel XIV kann in bekannter Weise durchgeführt werden, beispielsweise in einem inerten organischen Lösungsmittel, z.B. einem halogenierten Kohlenwasserstoff, wie Methylenchlorid, und in Gegenwart einer Base, z.B. eines Alkalimetallcarbonats, wie Natriumcarbonat, oder eines Alkalimetallbicarbonats, wie Natriumbicarbonat. Die Umsetzung erfolgt zweckmässigerweise bei Raumtemperatur.

Die Entfernung der Benzyl- und Benzyloxycarbonylgruppen aus einer Verbindung der Formel XV erfolgt nach bekannten Methoden, beispielsweise mittels Wasserstoff in Gegenwart eines Katalysators, wie eines Edelmetallkatalysators, z.B. Palladium auf Kohle, oder, falls $R^{20}$ von tert.Butyl verschieden ist, mittels Bromwasserstoff in Eisessig.

Die Cyclisation einer Säure der Formel XVI erfolgt nach bekannten Methoden. In einer bevorzugten Ausführungsform erfolgt die Cyclisation dadurch, dass man eine Verbindung der Formel XVI in bekannter Weise mit einem geeigneten Halogenierungsmittel, wie einem Phosphorpentahalogenid, z.B. Phosphorpentachlorid etc., oder einem Thionylhalogenid, z.B. Thionylchlorid etc., zum entsprechenden Säurehalogenid, z.B. Säurechlorid, umsetzt, das dann spontan zu einer Verbindung der Formel IV cyclisiert, worin $R^4$ und $R^5$ zusammen eine Oxogruppe und $Y^1$ $-CH_2-$ oder $-CH_2CH_2-$ bedeuten.

Die Reduktion einer Verbindung der Formel IV, worin $R^4$ und $R^5$ zusammen eine Oxogruppe und $Y^1$ $-CH_2-$ oder $-CH_2CH_2-$ bedeuten, erfolgt vorteilhafterweise mit Boran, zweckmässigerweise mit einem Borankomplex, wie Boran/Tetrahydrofuran, Boran/Dimethylsulfid, Boran/N,N-Diäthylanilin oder einem ähnlichen Komplex. Diese Reduktion mit Boran wird zweckmässigerweise in einem inerten organischen Lösungsmittel bei niedrigen Temperaturen durchgeführt, beispielsweise mit einem Boran/Tetrahydrofurankomplex in Tetrahydrofuran bei etwa 0°C bis etwa 20°C.

Die Verbindungen der Formel IV, worin $Y^1$ $-N(R^{60})-$bedeutet, können hergestellt werden, indem man beispielsweise eine Verbindung der obigen Formel XI mit einem Haloacetylhalogenid umsetzt, die erhaltene Verbindung der allgemeinen Formel

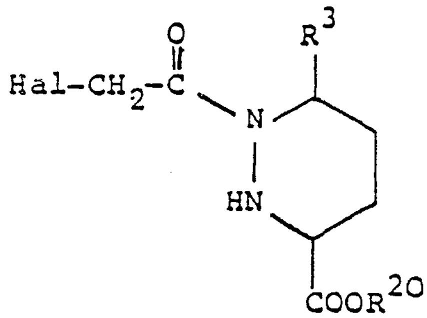

worin $R^3$, $R^{20}$ und Hal die oben angegebene Bedeutung besitzen, mit einer Verbindung der allgemeinen Formel

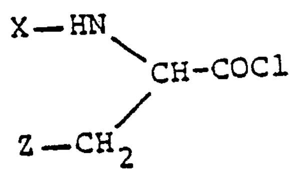

worin Z die oben angegebene Bedeutung besitzt und X eine Aminoschutzgruppe bedeutet, umsetzt, die mit X bezeichnete Aminoschutzgruppe in der erhaltenen Verbindung der allgemeinen Formel

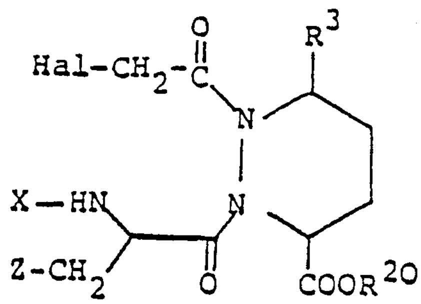

worin $R^3$, $R^{20}$, X, Z und Hal die oben angegebene Bedeutung besitzen, abspaltet, die so erhaltene Verbindung der allgemeinen Formel

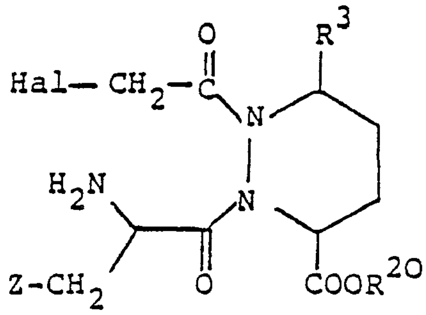

worin $R^3$, $R^{20}$, Z und Hal die oben angegebene Bedeutung besitzen, cyclisiert und die erhaltene Verbindung der allgemeinen Formel

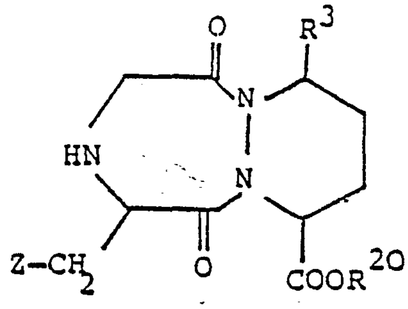

worin $R^3$, $R^{20}$ und Z die oben angegebene Bedeutung besitzen, mit einer Verbindung der Formel IX umsetzt. Erwünschtenfalls kann die erhaltene Verbindung der Formel IV, worin $R^4$ und $R^5$ zusammen eine Oxogruppe und $Y^1$ -N($R^{60}$)- bedeuten, zu einer entsprechenden Verbindung der Formel IV, worin $R^4$ und $R^5$ je Wasserstoff bedeuten, reduziert werden.

Die Umsetzung einer Verbindung der Formel XI mit einem Haloacetylhalogenid, wie Chloracetylchlorid, Bromacetylbromid, Bromacetylchlorid etc., kann nach bekannten Methoden durchgeführt werden. Beispielsweise wird die Reaktion in einem inerten organischen Lösungsmittel, wie einem halogenierten Kohlenwasserstoff, z.B. Methylenchlorid etc., und in Gegenwart einer Base, wie eines Alkalimetallcarbonates oder -bicarbonates, z.B. Natriumbicarbonat etc., durchgeführt. Zweckmässigerweise wird die Reaktion bei einer Temperatur zwischen etwa 0°C und der Raumtemperatur durchgeführt.

Als Aminoschutzgruppen X in Verbindung der Formel XVIII kommt jede konventionelle Aminoschutzgruppe in Frage. Vorzugsweise bedeutet X eine Aminoschutzgruppe, welche durch Basenbehandlung entfernt werden kann, wie die Trifluoracetylgruppe.

Die Umsetzung einer Verbindung der Formel XVII mit einer Verbindung der Formel XVIII erfolgt nach bekannten Methoden. Beispielsweise wird die Reaktion in einem inerten organi schen Lösungsmittel, wie einem halogenierten Kohlenwasserstoff, z.B. Methylenchlorid etc., und in Gegenwart einer Base, wie eines Alkalimetallcarbonates oder -bicarbonates, z.B. Natriumbicarbonat etc., durchgeführt. Die Reaktion erfolgt zweckmässigerweise bei einer Temperatur zwischen etwa 0°C und der Raumtemperatur.

Die Aminoschutzgruppe X in einer Verbindung der Formel XIX wird dann nach bekannten Methoden in Abhängigkeit von der Art der Schutzgruppe unter Bildung einer Verbindung der Formel XX abgespalten.

Die Cyclisation einer Verbindung der Formel XX kann beispielsweise leicht in Gegenwart einer Base, wie eines wässrigen Alkalimetallcarbonates oder -bicarbonates, z.B. Natriumbicarbonat etc., und in Gegenwart eines inerten organischen Lösungsmittels durchgeführt werden. Als inertes organisches Lösungsmittel kommt ein aliphatischer oder aromatischer Kohlenwasserstoff, z.B. n-Hexan, Benzol, Toluol etc., ein halogenierter Kohlenwasserstoff, z.B. Methylenchlorid, Chloroform, 1,1,1,-Trichloräthan etc., ein aliphatischer oder cyclischer Aether, z.B. Diäthyläther, Glykoldimethyläther. Tetrahydrofuran, Dioxan etc., ein Amid, z.B. Dimethylformamid etc., ein aliphatisches Keton, z.B. Aceton, Methyläthylketon etc., oder dergleichen in Frage. Obwohl die Cyclisation vorzugsweise bei ungefähr Raumtemperatur durchgeführt wird, kann sie erwünschtenfalls auch bei erhöhter Temperatur durchgeführt werden, beispielsweise bei einer Temperatur bis zur Rückflusstemperatur des Reaktionsgemisches.

Die weiter oben erwähnte Abspaltung der Aminoschutzgruppe X und die Cyclisation können erwünschtenfalls auch in umgekehrter Reihenfolge durchgeführt werden. Beispielsweise kann man eine Verbindung der Formel XIX cyclisieren, indem man diese mit einem Alkalimetallhydrid, wie Natriumhydrid, in einem inerten organischen Lösungsmittel, wie einem Amid, z.B. Dimethylformamid etc., bei ungefähr Raumtemperatur umsetzt und danach die Schutzgruppe X in bekannter Weise abspaltet.

Die Umsetzung einer Verbindung der Formel XXI mit einer Verbindung der Formel IX erfolgt in bekannter Weise. Die Umsetzung erfolgt zweckmässigerweise in Gegenwart von einem inerten organischen Lösungsmittel, wie beispielsweise einem halogenierten Kohlenwasserstoff, z.B. Methylenchlorid, Chloroform etc., einem Amid, z.B. Dimethylformamid etc., einem Nitril, z.B. Acetonitril etc., und dergleichen. Erwünschtenfalls kann man die Reaktion auch in Gegenwart von einer organischen Base, wie einem Trialkylamin, z.B. Triäthylamin, Diäthylisopropylamin etc., Pyridin,

N,N-Dimethylanilin etc., durchführen. Die Reaktion erfolgt bei ungefähr Raumtemperatur oder einer erhöhten Temperatur, beispielsweise bei der Rückflusstemperatur des Reaktionsgemisches.

Die Reduktion einer Verbindung der Formel IV, worin $R^4$ und $R^5$ zusammen eine Oxogruppe und $Y^1$ -N($R^{60}$)- bedeuten, wird vorteilhafterweise mit Boran durchgeführt, zweckmässigerweise mit einem Borankomplex, wie Boran/Tetrahydrofuran, Boran/Dimethylsulfid, Boran/N,N-Diäthylanilin oder einem ähnlichen Komplex. Diese Reduktion mit Boran erfolgt vorteilhafterweise in einem inerten organischen Lösungsmittel bei tiefer Temperatur, beispielsweise mit einem Boran/Tetrahydrofurankomplex in Tetrahydrofuran bei ungefähr 0°C bis ungefähr 20°C.

Gemäss einer Variante des oben beschriebenen Verfahrens zur Herstellung einer Verbindung der Formel IV, worin $Y^1$ -N($R^{60}$)- bedeutet, wird anstelle einer Verbindung der Formel XVIII eine entsprechende Verbindung verwendet, worin Z eine geschützte Hydroxygruppe bedeutet, beispielsweise Benzyloxy. Die geschützte Hydroxygruppe bleibt während der nachfolgenden Verfahrensschritte erhalten. Nach erfolgter Cyclisation wird die Hydroxyschutzgruppe in bekannter Weise abgespalten. Falls es sich bei der Hydroxyschutzgruppe um eine Benzyloxygruppe handelt, kann diese beispielsweise durch katalytische Hydrierung in Gegenwart eines Edelmetallkatalysators, wie Palladium, in die Hydroxygruppe übergeführt werden. Die erhaltene Verbindung, welche der Formel XXI entspricht, worin Z jedoch Hydroxy bedeutet, wird dann wie weiter oben beschrieben mit einer Verbindung der Formel IX umgesetzt und die erhaltene Verbindung in eine Verbindung der Formel IV, worin $R^4$ und $R^5$ zusammen eine Oxogruppe und $Y^1$ -N($R^{60}$)- bedeuten, übergeführt. Diese Ueberführung erfolgt in bekannter Weise beispielsweise durch Umsetzen mit einem Alkansulfonylhalogenid, wie Methansulfonylchlorid. Schliesslich kann man die erhaltene Verbindung der Formel IV erwünschtenfalls zur entsprechenden Verbindung, worin $R^4$ und $R^5$ je Wasserstoff bedeuten, in der oben beschriebenen Weise reduzieren.

Die in Verfahrensvariante c) als Ausgangsstoffe eingesetzten Verbindungen der Formel VI sind neu und ebenfalls Gegenstand der vorliegenden Erfindung.

Die Verbindungen der Formel VI können z.B. durch Umsetzen einer Verbindung der Formel XIII, worin Y $-CH_2-$oder $-CH_2CH_2-$ bedeutet, mit einer Verbindung der Formel V erhalten werden.

Die Umsetzung einer Verbindung der Formel XIII, worin Y $-CH_2-$ oder $-CH_2CH_2-$ bedeutet mit einer Verbindung der Formel V erfolgt nach bekannten Methoden. Die Reaktion kann in Gegenwart oder Abwesenheit eines inerten organischen Lösungsmittels durchgeführt werden. Falls ein inertes organisches Lösungsmittel verwendet wird, kommt beispielsweise ein aliphatischer oder aromatischer Kohlenwasserstoff, z.B. n-Hexan, Benzol, Toluol etc., ein halogenierter Kohlenwasserstoff, z.B. Methylenchlorid, Chloroform, 1,1,1-Trichloräthan etc., ein aliphatischer oder cyclischer Aether, z.B. Diäthyläther, Glykoldimethyläther, Tetrahydrofuran, Dioxan etc., ein Amid, z.B. Dimethylformamid, ein aliphatisches Keton, z.B. Aceton, Methyläthylketon etc., oder dergleichen in Frage. Als Lösungsmittel bevorzugt ist ein halogenierter Kohlenwasserstoff, insbesondere Methylenchlorid. Die Reaktionstemperatur ist nicht kritisch und kann zwischen ungefähr Raumtemperatur und der Rückflusstemperatur des Reaktionsgemisches liegen. In einer bevorzugten Ausführungsform erfolgt die Reaktion bei ungefähr Raumtemperatur.

Die in Verfahrensvariante d) als Ausgangsstoffe eingesetzten Verbindungen der Formel VII sind neu und ebenfalls Gegenstand der vorliegenden Erfindung.

Die Verbindungen der Formel VII können hergestellt werden, indem man beispielsweise eine Verbindung der obigen Formel X mit einer Verbindung der allgemeinen Formel

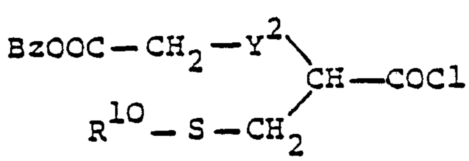

worin $R^{10}$, $Y^2$ und Bz die oben angegebene Bedeutung besitzen, umsetzt und die Benzyl- und Benzyloxycarbonylgruppen aus der erhaltenen Verbindung der allgemeinen Formel

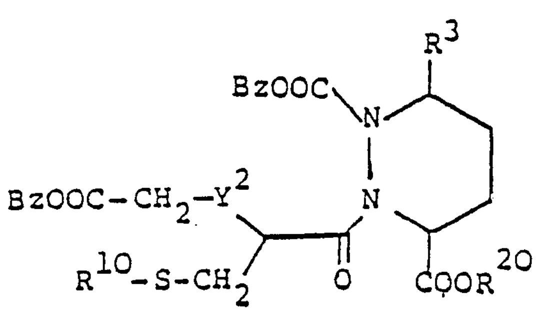

worin $R^3$, $R^{10}$, $R^{20}$, Y2 und Bz die oben angegebene Bedeutung besitzen, entfernt.

Die Umsetzung einer Verbindung der Formel X mit einer Verbindung der Formel XXII erfolgt nach an sich bekannten Methoden, beispielsweise in einem inerten organischen Lösungsmittel, z.B. einem halogenierten Kohlenwasserstoff, wie Methylenchlorid, in Gegenwart einer Base, z.B. eines Alkalimetallcarbonates, wie Natriumcarbonat, oder eines Alkalimetallbicarbonates, wie Natriumbicarbonat, zweckmässigerweise bei Raumtemperatur.

Die Entfernung der Benzyl- und Benzyloxycarbonylgruppen aus einer Verbindung der Formel XXIII erfolgt in bekannter Weise, beispielsweise mittels Wasserstoff in Gegenwart eines Katalysators, wie eines Edelmetallkatalysators, z.B. Palladium auf Kohle, oder, falls $R^{20}$ nicht tert.Butyl bedeutet, mittels Bromwasserstoff in Eisessig.

Die in Verfahrensvariante e) als Ausgangsstoffe eingesetzten Verbindungen der Formel VIII sind neu und ebenfalls Gegenstand der vorliegenden Erfindung.

Die Verbindungen der Formel VIII können hergestellt werden, indem man beispielsweise eine Verbindung der obigen Formel XVII mit einer Verbindung der allgemeinen Formel

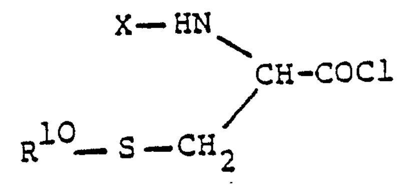

worin $R^{10}$ und X die oben angegebene Bedeutung besitzen, umsetzt und die Schutzgruppe X in der erhaltenen Verbindung der allgemeinen Formel

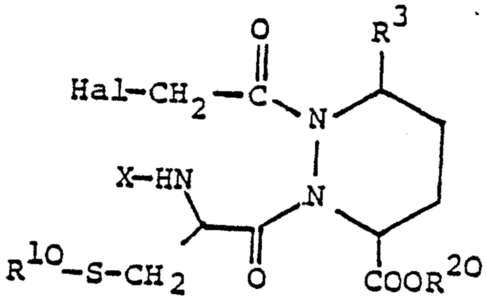

worin $R^3$, $R^{10}$, $R^{20}$, X und Hal die oben angegebene Bedeutung besitzen, abspaltet.

Die Umsetzung einer Verbindung der Formel XVII mit einer Verbindung der Formel XXIV erfolgt in bekannter Weise. Beispielsweise wird die Reaktion in einem inerten organischen Lösungsmittel, wie einem halogenierten Kohlenwasserstoff, z.B. Methylenchlorid etc., und in Gegenwart einer Base, wie eines Alkalimetallcarbonats oder -bicarbonats, z.B. Natriumbicarbonat etc., durchgeführt. Die Umsetzung erfolgt zweckmässig bei einer Temperatur zwischen etwa 0°C und der Raumtemperatur.

Die Abspaltung der Schutzgruppe X in einer Verbindung der Formel XXV erfolgt nach bekannten Methoden in Abhängigkeit von der Natur der Schutzgruppe.

Die in den Verfahrensvarianten a), b) und g) als Ausgangsstoffe eingesetzten Verbindungen der Formeln III, V und IX sind bekannt.

Die zur Herstellung der Ausgangsstoffe verwendeten Verbindungen der Formeln X, XII, XIV, XVIII, XXII und XXIV sind entweder bekannt oder Analoga bekannter Verbindungen, welche ihrerseits in ähnlicher Weise wie die bekannten Verbindungen hergestellt werden können.

Die Verbindungen der Formel I und deren pharmazeutisch verwendbare Salze sind wertvolle Antihypertensiva. Sie hemmen das Angiotensin umwandelnde Enzym (ACE), welches für die Umwandlung von Angiotensin I zu Angiotensin II verantwortlich ist, und sind deshalb nützlich, einen durch Angiotensin bewirkten Bluthochdruck zu vermindern oder zu lindern.

Die Aktivität der vorliegenden Verbindungen, das Angiotensin umwandelnde Enzym in vitro zu hemmen, kann durch den folgenden Test bestimmt werden.

Die verwendete Methode basiert auf einer Methode von Cushman und Cheung (Biochem. Pharmacol., 20, 1637-1648) unter Berücksichtigung der Modifikation von Hayakari et al. (Anal. Biochem. 84, 361-369). Das Substrat (Hippuryl-Histidyl-Leucin, 2 mM) wird in Abwesenheit oder Gegenwart verschiedener Konzentrationen der Testverbindungen in Kaliumphosphatpuffer (pH 8,3; 100 mM), welcher Natriumchlorid (300 mM) enthält, mit Angiotensin umwandelnden Enzym während 24 Minuten bei 37°C (Gesamtwert 500 µl) inkubiert. (Falls die Testsubstanz ein Ester ist, so ist es angezeigt, diesen mittels Schweineleberesterase vor Ausführung des Tests zu spalten). Die Reaktion wird durch Zugabe von 3 ml Kaliumphosphatpuffer (pH 8.3; 200 mM) bei 0°C abgebrochen. Man versetzt anschliessend mit 2,4,6-Trichlor-s-triazin (3%) in 1.5 ml Dioxan und schüttelt die erhaltene Mischung, bis der gelbe Chromophor voll entwickelt ist. Die Proben werden anschliessend zentrifugiert, um allenfalls entstandenen Festkörper abzutrennen. Der durch Reaktion von 2,4,6-Trichlor-s-triazin mit freier Hippursäure gebildete gelbe Chromophor wird spektrophotometrisch bei 382 nm ausgemessen. Der $IC_{50}$-Wert ist diejenige Konzentration einer Testverbindung, welche die Spaltung von Hippuryl-Histidyl-Leucin mittels Angiotensin umwandelndem Enzym unter den oben genannten Bedingungen um 50% reduziert.

Die nachfolgende Tabelle enthält die Resultate, welche mit repräsentativen Vertretern der Verbindungen der Formel I im vorhergehend beschriebenen Test erhalten wurden.

Tabelle

| Verbindung | $IC_{50}$ (nM) |
|---|---|
| A | 4,0 |
| B | 2,6 |
| C | 19 |
| D | 51 |

Verbindung A: Octahydro-9(S)-mercaptomethyl-6,10-dioxo-6H-pyridazo[1,2-a][1,2]diazepin-1(S)-carbonsäure

Verbindung B: Octahydro-9(S)-mercaptomethyl-10-oxo-6H-pyridazo[1,2-a][1,2]diazepin-1(S)-carbonsäure

Verbindung C: Decahydro-10(R,S)-mercaptomethyl-6,11-dioxo-6H-pyridazo[1,2-a][1,2-]diazepin1(S)-carbonsäure

Verbindung D: Octahydro-9-mercaptomethyl-8-methyl-6,10-dioxo-6H-pyridazo[1,2-a][-1,2,5]triazepin-1(S)-carbonsäure (Diastereoisomer 1).

Die Verbindungen der Formel I und deren pharmazeutisch verwendbare Salze können in Form pharmazeutischer Präparate, welche diese Verbindungen zusammen mit geeigneten pharmazeutischen Trägerstoffen enthalten, als Arzneimittel verwendet werden. Als Trägerstoffe kommen dabei für die enterale, beispielsweise orale, oder parenterale Verabreichung geeignete organische oder anorganische Trägerstoffe in Frage, beispielsweise Wasser, Gelatine, Gummi arabicum, Lactose, Stärke, Magnesiumsstearat, Talk, pflanzliche Oele, Polyalkylenglykole, Vaseline und dergleichen. Die pharmazeutischen Präparate können in fester Form, z.B. als Tabletten, Dragées, Suppositorien oder Kapseln, oder in flüssiger Form, z.B. als Lösungen, Suspensionen oder Emulsionen, verarbeitet werden. Die pharmazeutischen Präparate können üblichen pharmazeutischen Behandlungen, wie Sterilisieren, unterworfen werden und/oder Zusatzstoffe, wie Konservierungsmittel, Stabilisierungsmittel, Netzmittel oder Emulgiermittel, Salze zum Variieren des osmotischen Druckes oder Puffer enthalten. Die pharmazeutischen Präparate können auch noch andere therapeutisch wertvolle Verbindungen enthalten.

Die Verbindungen der Formel I und deren pharmazeutisch verwendbare Salze können Erwachsenen in täglichen Dosen von etwa 0,1 bis 100 mg, vorzugsweise etwa 1 bis 50 mg, pro kg Körpergewicht verabreicht werden. Die tägliche Dosis kann in einer einzigen Dosis oder Teildosen verabreicht werden. Es sei an dieser Stelle betont, dass die oben genannten Dosierungsbereiche nur als Beispiele angegeben sind, und dass sie nach oben oder unten variiert werden können, in Abhängigkeit von Faktoren, wie den spezifischen Eigenschaften der zu verabreichenden Verbindung oder des zu verabreichenden Salzes, der Art der Verabreichung, der Schwere der zu behandelnden Krankheit und dem Zustand des Patienten, wie er vom behandelnden Arzt festgestellt wird.

Die folgenden Beispiele veranschaulichen die vorliegende Erfindung.

Beispiel 1

2,0 g (6,8 mMol) tert.Butyl octahydro-9-methylen-6,10-dioxo-6H-pyridazo[1,2-a][1,2]diazepin-(1S)-carboxylat und 1,0 ml (14 mMol) Thiolessigsäure in 20 ml Methylenchlorid werden während 20 Stunden bei Raumtemperatur gerührt. Nach dem Eindampfen wird das verbleibende Oel an Silicagel mit Aethylacetat/n-Hexan als Eluierungsmittel chromatographiert. Es werden zunächst 1,3 g (52%) tert.Butyl 9(S)-acetylthio methyl-octahydro-6,10-dioxo-6H-pyridazo[1,2-a][1,2]diazepin-1(S)-carboxylat als weisser Festkörper vom Schmelzpunkt 95-96°C (aus Diäthyläther/n-Hexan) und danach 1,16 g (46%) tert.Butyl 9(R)-acetylthiomethyl-octahydro-6,10-dioxo-6H-pyridazo[1,2-a][1,2]diazepin-1(S)-carboxylat als amorpher weisser Festkörper erhalten.

Das als Ausgangsmaterial eingesetzte tert.Butyl octahydro-9-methylen-6,10-dioxo-6H-pyridazo[1,2-a][1,2]diazepin-1(S)-carboxylat wurde wie folgt hergestellt:

Eine Lösung von 10 g (0,0313 Mol) 1-Benzyloxycarbonyl-hexahydro-pyridazin-3(S)-carbonsäure-tert.butylester in 100 ml Methanol wird bei Raumtemperatur und Atmosphärendruck über 5%igem Palladium auf Kohle hydriert. Der Katalysator wird abfiltriert, und das Filtrat zur Trockene eingedampft. Der zurückbleibende Hexahydro-pyridazin-3(S)-carbonsäure-tert.butylester wird in 100 ml Dioxan aufgenommen, und die auf 0°C abgekühlte Lösung mit einer Lösung von 3,94 g (0,0313 Mol) α-Methylen-glutarsäureanhydrid in 100 ml Dioxan versetzt. Das Gemisch wird 18 Stunden bei 20°C gerührt, und das Lösungsmittel durch Abdampfen entfernt. Der Rückstand wird zwischen Methyl-tert.butyläther und gesättigter Natriumbicarbonatlösung verteilt. Die wässrige Phase wird mit Salzsäure angesäuert und mit Methylenchlorid extrahiert,

wobei man 8,34 g (85%) 3(S)-tert.Butoxycarbonyl-hexahydro-α-methylen-δ-oxo - 1-pyridazinpentansäure in Form von weissen Kristallen erhält, Schmelzpunkt 96-99°C. 5,0 g (16 mMol) dieser Säure werden in 350 ml Tetrahydrofuran aufgenommen, und die Lösung auf 0°C gekühlt. 3,75 g (18 mMol) Phosphorpentachlorid werden zugegeben, und das Gemisch 1 Stunde bei 0°C und 18 Stunden bei 20°C gerührt. Das Lösungsmittel wird abgedampft, und der Rückstand zwischen Aethylacetat und gesättigter Natriumbicarbonatlösung verteilt. Die organische phase wird eingedampft, und der Rückstand an Silicagel mit Aehylacetat/n-Hexan als Eluierungsmittel chromatograhiert. Es werden dabei 3,7 g (79%) tert. Butyl octahydro-9-methylen-6,10-dioxo-6H - pyridazo[1,2-a][1,2]diazepin-1(S)-carboxylat als weisser Festkörper erhalten, Schmelzpunkt 105-106°C (aus Hexan).

Beispiel 2

370 mg (1 mMol) tert.Butyl 9(S)-acetylthiomethyl-octahydro-6,10-dioxo-6H - pyridazo[1,2-a][1,2]diazepin-1(S)-carboxylat werden in 5 ml Tetrahydrofuran gelöst, und die Lösung bei 0°C mit 2 ml 0,5M Boran/Tetrahydrfuran versetzt. Das Gemisch wird 1 Stunde bei 0°C und weitere 3 Stunden bei 20°C gerührt und danach mit Methylenchlorid verdünnt. 15 ml verdünnte Salzsäure werden vorsichtig zugegeben, und das Reaktionsgemisch danach eine halbe Stunde bei 0°C gerührt. Mit Natriumcarbonat wird danach der pH-Wert des Reaktionsgemisch auf 8 eingestellt, und die beiden Phasen getrennt. Die organische Phase wird eingedampft, und der Rückstand an Silicagel mit Aethylacetat/n-Hexan als Eluierungsmittel chromatographiert. Dabei werden 150 mg (42%) tert.Butyl 9(S)-acetylthiomethyl-octahydro-10-oxo-6H - pyridazo[1,2-a][1,2]diazepin-1(S)-carboxylat als farbloses Oel erhalten.

Beispiel 3

In analoger Weise wie in Beispiel 2 beschrieben, wurden aus 370 mg (1 mMol) tert.Butyl 9(R)-acetylthiomethyl-octahydro-6,10-dioxo-6H - pyridazo[1,2-a][1,2]diazepin-1(S)-carboxylat 200 mg (56%) tert.Butyl 9(R)-acetylthiomethyl-octahydro-10-oxo-6H - pyridazo[1,2-a][1,2]diazepin-1(S)-carboxylat als farbloses Oel erhalten.

Beispiel 4

300 mg (0,81 mMol) tert.Butyl 9(S)-acetylhiomethyl-octahydro-6,10-dioxo-6H - pyridazo[1,2-a][1,2]diazepin-1(S)-carboxylat werden in 3 ml Trifluoressigsäure gelöst, und die Lösung bei 20°C 1,5 Stunden stehengelassen. Durch Eindampfen erhält man 230 mg (90%) 9(S)-Acetylthiomethyl-octahydro-6,10-dioxo-6H - pyridazo[1,2-a][1,2]pyridazin-1(S)-carbonsäure als weissen Festkörper, Schmelzpunkt 133-135°C (aus Aethylacetat/n-Hexan).

Beispiel 5

In analoger Weise wie in Beispiel 4 beschrieben, wurden aus 300 mg (0,81 mMol) tert.Butyl 9(R)-acetylthiomethyl-octahydro-6,10-dioxo-6H - pyridazo[1,2-a][1,2]diazepin-1(S)-carboxylat 235 mg 9(R)-Acetylthiomethyl-octahydro-6,10-dioxo-6H - pyridazo[1,2-a][1,2]diazepin-1(S)-carbonsäure als amorpher Weisser Festkörper erhalten.

Beispiel 6

In analoger Weise wie in Beispiel 4 beschrieben, wurden aus 130 mg tert.Butyl 9(S)-acetylthiomethyl-octahydro-10-oxo-6H - pyridazo[1,2-a][1,2]diazepin-1(S)-carboxylat 105 mg (S)-Acethylthiomethyl-octahydro-10-oxo-6H - pyridazo[1,2-a][1,2]diazepin-1(S)-carbonsäure als weisser Schaum erhalten.

Beispiel 7

In analoger Weise wie in Beispiel 4 beschrieben, wurden aus 180 mg tert.Butyl 9(R)-acetylthiomethyl-octahydro-10-oxo-6H - pyridazo[1,2-a][1,2]diazepin-1(S)-carboxylat 150 mg 9(R)-Acetylthiomethyl-octahydro-10-oxo-6H - pyridazo[1,2-a][1,2]diazepin-1(S)-carbonsäure als farbloser Gummi erhalten.

Beispiel 8

160 mg (0,51 mMol) 9(S)-Acetylthiomethyl-octahydro-6,10-dioxo-6H - pyridazo[1,2-a][1,2]diazepin-1(S)-carbonsäure werden in 1,6 ml Wasser enthaltend 1,6 ml konz. Ammoniumhydroxid gelöst, und die Mischung 1 Stunde bei 20°C stehengelassen. Das Gemisch wird danach mit Salzsäure auf pH 1 ein gestellt und mit Methylenchlorid extrahiert. Die Methylenchloridextrakte werden eingedampft, und der Rückstand mit n-Hexan angerieben, wobei man 116 mg Octahydro-9(S)-mercaptomethyl-6,10-dioxo-6H - pyridazo[1,2-a][1,2]diazepin-1(S)-carbonsäure als amorphen Festkörper erhält.

Beispiel 9

In analoger Weise wie in Beispiel 8 beschrieben, wurden aus 210 mg 9(R)-Acetylthiomethyl-octahydro-6,10-dioxo-6H - pyridazo[1,2-a][1,2]diazepin-1(S)-carbonsäure 75 mg Octahydro-9(R)-mercaptomethyl-6,10-dioxo-6H - pyridazo[1,2-a][1,2]diazepin-1(S)-carbonsäure als amorpher Festkörper erhalten.

Beispiel 10

In analoger Weise wie in Beispiel 8 beschrieben, wurden aus 110 mg 9(S)-Acetylthiomethyl-octahydro-10-oxo-6H - pyridazo[1,2-a][1,2]diazepin-1(S)-carbonsäure 75 mg Octahydro-9(S)-mercaptomethyl-10-oxo-6H-pyridazo[1,2-a]-[1,2] diazepin-1(S)-carbonsäure als amorpher Festkörper erhalten.

Beispiel 11

In analoger Weise wie in Beispiel 8 beschrieben, wurden aus 165 mg 9(R)-Acetylthiomethyl-octahydro-10-oxo-6H - pyridazo[1,2-a][1,2]diazepin-1(S)-carbonsäure 100 mg Octahydro-9(R)-mercaptomethyl-10-oxo-6H - pyridazo[1,2-a][1,2]diazepin-1(S)-carbonsäure als amorpher Festkörper erhalten.

Beispiel 12

In analoger Weise wie in Beispiel 1 beschrieben, wurden aus 0,36 g tert.Butyl decahydro-10-methylen-6,11-dioxo-6H - pyridazo[1,2-a][1,2]diazocin-1(S)-carboxylat 0,21 g tert.Butyl 10(R,S)-acetylthiomethyl-decahydro-6,11-dioxo-6H -pyridazo[1,2-a][1,2]diazocin-1(S)-carboxylat in Form eines weissen Festkörpers erhalten, Schmelzpunkt 105-108°C (aus Methyl-tert.butyläther).

Das als Ausgangsmaterial eingesetzte tert.Butyl decahydro-10-methylen-6,11-dioxo-6H - pyridazo[1,2-a][1,2]diazocin-1(S)-carboxylat wurde wie folgt hergestellt:

Eine Lösung von 1,86 g des 1-Monoäthylesters der 2-Methylenadipinsäure in 25 ml wasserfreiem Tetrahydrofuran wird auf -10°C gekühlt und unter Rühren mit 1,01 g Triäthylamin und 1,37 g Isobutylchloroformat versetzt. Das Reaktionsgemisch wird 15 Minuten bei 10°C gerührt, und eine Lösung von 1,86 g Hexahydro-pyridazin-3-carbonsäure-tert.butylester in 25 ml wasserfreiem Tetrahydrofuran werden dann zugegeben. Das Reaktionsgemisch wird 30 Minuten bei -10°C und danach 16 Stunden bei Raumtemperatur gerührt. Das Lösungsmittel wird abgedampft, und der Rückstand zwischen Aethylacetat und 1N wässriger Salzsäure verteilt. Die organische Phase wird mit gesättigter Natriumchloridlösung und gesättigter wässriger Natriumbicarbonatlösung gewaschen und danach eingedampft, wobei man 1,99 g tert.Butyl-1-[5-(äthoxycarbonyl)-5-hexenoyl]-hexahydro - 3(S)-pyridazincarboxylat in Form eines weissen Festkörpers erhält, Schmelzpunkt 63-65°C (aus Aethylacetat/n-Hexan).

Eine Lösung von 8,2 g tert.Butyl 1-[5-(äthoxycarbonyl)-5-hexenoyl]-hexahydro - 3(S)-pyridazincarboxylat in 25 ml Aethanol wird mit 23,7 ml einer 1N wässrigen Natriumhydroxidlösung 16 Stunden umgesetzt. Die Lösung wird mit 80 ml Wasser verdünnt, und das Volumen danach durch Abdampfen auf 80 ml reduziert. Die Lösung wird mit Diäthyläther gewaschen und der pH-Wert der wässrigen Lösung wird mit 2N wässriger Salzsäure auf 3 eingestellt. Die Lösung wird dann mit Methylenchlorid extrahiert. Die organische Lösung wird eingedampft, und der Rückstand an Silicagel mit Methylenchlorid/Methanol (9:1) als Eluierungsmittel chromatographiert. Dabei werden 3,62 g tert.Butyl 1-[5-(carboxy)-5-hexenoyl]-hexa hydro - 3(S)-pyridazincarboxylat in Form eines weissen Festkörpers erhalten, Schmelzpunkt 86-88°C (aus n-Hexan).

Eine Lösung von 0,56 g tert.Butyl 1-[5-carboxy)-5-hexenoyl]-hexahydro - 3(S)-pyridazincarboxylat in 12 ml Methylenchlorid wird mit 0,31 g Thionylchlorid versetzt, und die erhaltene Lösung bei Raumtemperatur unter einem schwachen Strom von Stickstoff 20 Stunden gerührt. Die Lösung wird danach mit gesättigter wässriger Natriumbicarbonatlösung gewaschen und eingedampft. Der Rückstand wird an Silicagel mit Diäthyläther als Eluierungsmittel chromatographiert. Dabei werden 0,21 g tert.Butyl decahydro-10-methylen-6,11-dioxo-6H - pyridazo[1,2-a][1,2]diazocin-1(S)-carboxylat in Form eines weissen Festkörpers erhalten, Schmelzpunkt 108-110°C (aus Diäthyläther).

Beispiel 13

0,19 g tert.Butyl 10(R,S)-acetylthiomethyl-decahydro-6,11-dioxo-6H - pyridazo[1,2-a][1,2]diazocin-1(S)-carboxylat werden mit 2 ml Trifluoressigsäure bei Raumtemperatur 2 Stunden umgesetzt. Das Gemisch wird danach eingedampft, und der Rückstand in Toluol aufgenommen und eingedampft. Dieses Prozedere wird zweimal wiederholt. Der Rückstand wird unter Stickstoff mit 1,5 ml konz. wässrigem Ammoniak und 1,5 ml Wasser bei Raumtemperatur 1,5 Stunden gerührt. Die Lösung wird auf 0°C abgekühlt, und der pH-Wert mit konz. Salzsäure auf 1 eingestellt. Die Lösung wird mit Kochsalz gesättigt und mit Methylenchlorid extrahiert. Die organische Lösung wird eingedampft, wobei man 75 mg Decahydro-10(R,S)-mercaptomethyl-6,11-dioxo-6H - pyridazo[1,2-a][1,2]diazocin-1(S)-carbonsäure als amorphen weissen Festkörper erhält.

Beispiel 14

720 mg (1,5 mMol) tert.Butyl 8-benzyl-octahydro-9-methansulfonoxymethyl-6,10 - dioxo-6H-pyridazo[1,2-a] [1,2,5]triazepin-1(S)-carboxylat (Diastereoisomer 1) werden in 20 ml Butan-2-on gelöst, und die Lösung mit 340 mg (2 mMol) Kaliumthiolacetat versetzt. Das Reaktionsgemisch wird 8 Stunden zum Rückfluss erhitzt und danach zur Trockene eingedampft. Der Rückstand wird zwischen Aethylacetat und Wasser verteilt. Die organische Phase wird nacheinander mit wässriger Natriumbicarbonatlösung und gesättigter Kochsalzlösung gewaschen, über wasserfreiem Natriumsulfat getrocknet und eingedampft. Der Rückstand wird an Silicagel mit Aethylacetat/n-Hexan als Eluierungsmittel chromatographiert. Dabei werden 400 mg tert.Butyl 9-acetylthiomethyl-8-benzyl-octahydro-6,10-dioxo - 6H-pyridazo[1,2-a][1,2,5]triazepin-1(S)-carboxylat (Diastereoisomer 1) in Form gelber Kristalle erhalten, Schmelzpunkt 156-158°C (aus Aethylacetat/n-Hexan).

Das als Ausgangsmaterial eingesetzte tert.Butyl 8-benzyl-octahydro-9-methansulfonyloxymethyl-6,10 - dioxo-6H-pyridazo[1,2-a][1,2,5]triazepin-1(S)-carboxylat (Diastereoisomer 1) wurde wie folgt hergestellt:

Eine Lösung von 20 g (0,062 Mol) 1-Benzyloxycarbonyl-hexahydro-pyridazin - 3(S)-carbonsäure-tert.butylester in 400 ml Aethanol wird während 5 Stunden bei Atmosphärendruck über 2 g

5%igem Palladium auf Kohle hydriert. Der Katalysator wird abfiltriert, und das Filtrat eingedampft. Der Rückstand wird mit einer Mischung von 50 ml Methylenchlorid und 75 ml wässriger Natriumbicarbonatlösung bei 0°C gerührt, während man über einen Zeitraum von 15 Minuten 5,16 ml Bromacetylchlorid in 150 ml Methylenchlorid gelöst zugibt. Das Reaktionsgemisch wird 45 Minuten weitergerührt, und danach werden die beiden phasen getrennt. Die organische Phase wird über wasserfreiem Natriumsulfat getrocknet und eingedampft. Der Rückstand wird an Silicagel mit Aethylacetat/n-Hexan als Eluierungsmittel chromatographiert. Dabei werden 9,9 g tert.Butyl 1-bromacetyl-hexahydro -3(S)-pyridazincarboxylat in Form eines weissen Festkörpers erhalten, Schmelzpunkt 99-101°C (aus Aethylacetat/n-Hexan).

13,9 g (0,048 Mol) N-Trifluoracetyl-O-benzyl-D,L-serin werden in 150 ml Methylenchlorid gelöst, und die Lösung auf -20°C gekühlt. Man versetzt die Lösung bei dieser Temperatur mit 9,96 g (0,048 Mol) Phosphorpentachlorid, lässt auf Raumtemperatur erwärmen und rührt während 30 Minuten bei Raumtemperatur. Nach dem Abdampfen wird ein öliger Rückstand erhalten, welcher in 150 ml Methylenchlorid gelöst wird. Die Lösung wird unter Rühren innerhalb 1 Stunde bei 0°C zu einer Mischung von 9,8 g tert.Butyl 1-bromacetyl-hexahydro-3(S)-pyridazincarboxylat, 100 ml Methylenchlorid und 100 ml wässriger Natriumbicarbonatlösung gegeben. Das Gemisch wird 17 Stunden bei Raumtemperatur gerührt, und die Phasen werden danach getrennt. Die organische Phase wird über wasserfreiem Natriumsulfat getrocknet und eingedampft. Der Rückstand wird an Silicagel mit Aethylacetat/n-Hexan als Eluierungsmittel chromatographiert, wobei man 12,5 g tert.Butyl 1-bromacetyl-2-{[3-benyloxy-2(R,S) - trifluoracetylamino]propanoyl}-pyridazin-3(S)-carboxylat in Form eines gelben Oels erhält.

Das obige Oel wird in 650 ml trockenem Dimethylformamid gelöst, und die Lösung mit 980 mg Natriumhydrid versetzt. Nach 2 Stunden bei 20°C wird die Lösung auf 1,6 l Eiswasser gegossen und mit Methylenchlorid extrahiert. Der organische Extrakt wird eingedampft, und der Rückstand an Silicagel mit Aethylacetat/n-Hexan als Eluierungsmittel chromatographiert, wobei man zwei Diastereoisomere erhält, nämlich 3,1 g (Diastereoisomer 1) und 1,05 g (Diastereoisomer 2) von tert.Butyl 9-benzyloxymethyl-octahydro-6,10-dioxo-6H - pyridazo[1,2-a][1,2,5]triazepin-1(S)-carboxylat in Form von leicht gelben Gummis.

500 mg des Diastereoisomeren 1 werden in 10 ml Methanol gelöst, welches wenige Tropfen Essigsäure enthält. Die Lösung wird dann bei Atmosphärendruck über 50 mg 5%igem Palladium auf Kohle 17 Stunden hydriert. Der Katalysator wird abfiltriert, und das Filtrat eingedampft, wobei man 440 mg tert.Butyl octahydro-9-hydroxymethyl-6,10-dioxo-6H - pyridazo[1,2-a][1,2,5]triazepin-1(S)-carboxylat (Diastereoisomer 1) in Form eines farblosen Gummis erhält.

Der obige Gummi wird mit 0,41 ml Benzylbromid in 20 ml Acetonitril, welches 1 ml Diäthylisopropylamin enthält, bei 20°C während 24 Stunden umgesetzt. Nach Eindampfen und Chromatographie des Rückstandes an Silicagel mit Aethylacetat/n-Hexan als Eluierungsmittel erhält man 400 mg tert.Butyl 8-benzyl-octahydro-9-hydroxymethyl-6,10-dioxo-6H - pyridazo[1,2-a][1,2,5]triazepin-1(S)-carboxylat (Diastereoisomer 1) in Form eines weissen Festkörpers, Schmelzpunkt 133-135°C (aus Aethylacetat/n-Hexan).

265 mg tert.Butyl 8-benzyl-octahydro-9-hydroxymethyl-6,10-dioxo-6H - pyridazo[1,2-a][1,2,5]triazepin-1(S)-carboxylat (Diastereoisomer 1) in 10 ml Methylenchlorid werden bei 20°C mit 0,2 ml Triäthylamin und danach mit 0.09 ml Methansulfonylchlorid versetzt. Das Reaktionsgemisch wird 2 Stunden bei Raumtemperatur gerührt, mit Wasser gewaschen, über wasserfreiem Natriumsulfat getrocknet und eingedampft, wobei man 230 mg tert.Butyl 8-benzyl-octahydro-9-methansulfonyloxymethyl-6,10 - dioxo-6H-pyridazo[1,2-a][1,2,5]triazepin-1(S)-carboxylat (Diastereoisomer 1) in Form eines weissen Festkörpers erhält, Schmelzpunkt 55-57°C (aus Diäthyläther/n-Hexan).

Beispiel 15

110 mg tert.Butyl 9-acetylthiomethyl-8-benzyl-octahydro-6,10-dioxo - 6H-pyridazo[1,2-a][1,2,5]triazepin-1(S)-carboxylat (Diastereoisomer 1) werden mit 0,5 ml 45%igem Bromwasserstoff in Essigsäure während 30 Minuten bei 20°C umgesetzt. Dann wird wasserfreier Diäthyläther zugegeben, und der Niederschlag abfiltriert, wobei man 60 mg amorphes 9-Acetylthiomethyl-8-benzyl-octahydro-6,10-dioxo-6H -pyri dazo[1,2-a][1,2,5]triazepin-1(S)-carbonsäure-hydrobromid (Diastereoisomer 1) erhält.

Beispiel 16

In analoger Weise wie in Beispiel 8 beschrieben, wurden aus 60 mg 9-Acetylthiomethyl-8-benzyl-octahydro-6,10 -dioxo-6H-pyridazo[1,2-a][1,2,5]triazepin-hydrobromid nach Chromatographie an Silicagel mit 2% Essigsäure in Diäthyläther als Eluierungsmittel 20 mg 8-Benzyl-octahydro-9-mercaptomethyl-6,10-dioxo-6H - pyridazo[1,2-a][1,2,5]triazepin-1(S)-carbonsäure (Diastereoisomer 1) in Form eines weissen Festkörpers erhalten, Schmelzpunkt 85°C (Zers.; aus Diäthyläther/n-Hexan).

Beispiel 17

In analoger Weise wie in Beispiel 14 beschrieben, wurden aus tert.Butyl 8-methyl-octahydro-9-methansulfonyloxymethyl-6,10 - dioxo-6H-pyridazo[1,2-a][1,2,5]triazepin-1(S)-carboxylat zwei Diastereoisomere (Diastereoisomer 1 und Diastereoisomer 2) von tert.Butyl 9-acetylthiomethyl-8-methyl-octahydro-6,10-dioxo - 6H-pyridazo[1,2-a][1,2,5]triazepin-1(S)-carboxylat in Form von Gummis erhalten.

Das als Ausgangsmaterial eingesetzte tert.Butyl 8-methyl-octahydro-9-methansulfonyloxymethyl-6,10 - dioxo-6H-pyridazo[1,2-a][1,2,5]triazepin-1(S)-carboxylat wurde in analoger Weise wie in Beispiel 14 für die Herstellung des dort verwendeten Ausgangsmaterials beschrieben hergestellt und in Form eines Gummis erhalten.

Beispiel 18

In analoger Weise wie in Beispiel 15 beschrieben, wurden aus 110 mg tert.Butyl 9-acetylthiomethyl-8-methyl-octahydro-6,10-dioxo-6H - pyridazo[1,2-a][1,2,5]triazepin-1(S)-car boxylat

(Diastereoisomer 1) 100 mg 9-Acetylthiomethyl-8-methyl-octahydro-6,10-dioxo-6H - pyridazo[1,2-a][1,2,5]triazepin-1(S)-carbonsäure (Diastereoisomer 1) in Form eines amorphen Festkörpers erhalten.

Beispiel 19

In analoger Weise wie in Beispiel 15 beschrieben, wurden aus 80 m, tert.Butyl 9-acetylthiomethyl-8-methyl-octahydro-6,10-dioxo - 6H-pyridazo[1,2-a][1,2,5]triazepin-1(S)-carboxylat (Diastereoisomer 2) 50 mg 9-Acetylthiomethyl-8-methyl-octahydro-6,10-dioxo-6H - pyridazo[1,2-a][1,2,5]triazepin-1(S)-carbonsäure (Diastereoisomer 2) in Form eines amorphen Festkörpers erhalten.

Beispiel 20

In analoger Weise wie in Beispiel 8 beschrieben, wurden aus 50 mg 9-Acetylthiomethyl-8-methyl-octahydro-6,10-dioxo-6H - pyridazo[1,2-a][1,2,5]triazepin-1(S)-carbonsäure (Diastereoisomer 1) 10 mg Octahydro-9-mercaptomethyl-8-methyl-6,10-dioxo-6H - pyridazo[1,2-a][1,2,5]triazepin-1(S)-carbonsäure (Diastereoisomer 1) in Form eines weissen Lyophilisates erhalten.

Beispiel 21

In analoger Weise wie in Beispiel 8 beschrieben, wurden aus 50 mg 9-Acetylthiomethyl-8-methyl-octahydro-6,10-dioxo-6H - pyridazo[1,2-a][1,2,5]triazepin-1(S)-carbonsäure (Diastereoisomer 2) 10 mg Octahydro-9-mercaptomethyl-8-methyl-6,10-dioxo-6H - pyridazo[1,2-a][1,2,5]triazepin-1(S)-carbonsäure (Diastereoisomer 2) in Form eines weissen Lyophilisates erhalten.

Beispiel 22

In analoger Weise wie in Beispiel 1 beschrieben, wurden aus 2,02 g Methyl octahydro-9-methylen-6,10-dioxo-6H -pyridazo[1,2-a][1,2]diazepin-1(S)-carboxylat 1,17 g Methyl 9(S)-acetylthiomethyl-octahydro-6,10-dioxo-6H - pyridazo[1,2-a][1,2]diazepin-1(S)-carboxylat in Form weisser Kristalle, Schmelzpunkt 127-129°C (aus Diäthyläther/n-Hexan) und 1,2 g Methyl 9(R)-acetylthiomethyl-octahydro-6,10-dioxo-6H - pyridazo[1,2-a][1,2]diazepin-1(S)-carboxylat in Form eines farblosen Gummis erhalten.

Das als Ausgangsmaterial eingesetzte Methyl octahydro-9-methylen-6,10-dioxo-6H - pyridazo[1,2-a][1,2]diazepin-1(S)-carboxylat wurde wie folgt hergestellt:

10 g tert.Butyl octahydro-9-methylen-6,10-dioxo-6H - pyridazo[1,2-a][1,2]diazepin-1(S)-carboxylat (aus Beispiel 1) werden mit 40 ml Trifluoressigsäure 3 Stunden bei 20°C gerührt. Das Gemisch wird dann eingedampft, und das erhaltene Oel mit Diäthyläther versetzt, wobei man 7,6 g Octahydro-9-methylen-6,10-dioxo-6H - pyridazo[1,2-a][1,2]diazepin-1(S)-carbonsäure in Form eines weissen Festkörpers erhält, Schmelzpunkt 169-172°C.

7,6 g Octahydro-9-methylen-6,10-dioxo-6H - pyridazo[1,2-a][1,2]diazepin-1(S)-carbonsäure werden in 200 ml Aethylacetat suspendiert, und die Suspension während der Zugabe von 100 ml ätherischer Diazomethanlösung bei 0°C gerührt. Nach 30 Minuten wird das überschüssige Diazomethan durch tropfenweise Zugabe von Essigsäure zerstört. Das Reaktionsgemisch wird mit wässriger Natriumbicarbonatlösung gewaschen, über Natriumsulfat getrocknet und eingedampft, wobei man 5,17 g Methyl octahydro-9-methylen-6,10-dioxo-6H - pyridazo[1,2-a][1,2]diazepin-1(S,-carboxylat in Form eines weissen Festkörpers erhält, Schmelzpunkt 73-75°C (aus n-Hexan).

Die folgenden Beispiele veranschaulichen pharmazeutische Präparate, enthaltend eine erfindungsgemässe Verbindung:

Beispiel A

Tabletten, enthaltend die folgenden Bestandteile, können in herkömmlicher Weise hergestellt werden:

| Bestandteile | | pro Tablette |
|---|---|---|
| Verbindung der Formel | | 10,0 mg |
| Lactose | | 125,0 mg |
| Maisstärke | | 75,0 mg |
| Talk | | 4,0 mg |
| Magnesiumstearat | | 1,0 mg |
| | Totelgewicht | 215,0 mg |

Beispiel B

Kapseln, enthaltend die folgenden Bestandteile, können in herkömmlicher Weise hergestellt werden:

| Bestandteile | | pro Kapsel |
|---|---|---|
| Verbindung der Formel I | | 25,0 mg |
| Lactose | | 150,0 mg |
| Maisstärke | | 20,0 mg |
| Talk | | 5,0 mg |
| | Kapselfüllgewicht | 200,0 mg |

**Ansprüche**

1. Verbindungen der allgemeinen Formel

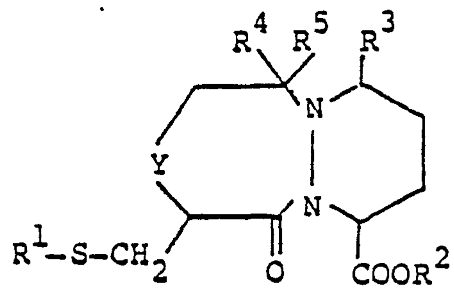

worin $R^1$ Wasserstoff, Alkanoyl oder Aroyl, $R^2$ Wasserstoff oder Alkyl, $R^3$ Wasserstoff oder Aryl, $R^4$ und $R^5$ je Wasserstoff oder zusammen eine Oxogruppe und Y $-CH_2-$, $-CH_2CH_2-$ oder $-N(R^6)-$ bedeuten, worin $R^6$ Wasserstoff, Alkyl oder Aralkyl bedeutet, sowie pharmazeutisch verwendbare Salze davon mit Basen, falls $R^2$ Wasserstoff bedeutet, und pharmazeutisch verwendbare Salze davon mit Säuren, falls Y-$N(R^6)$- bedeutet.

2. Verbindungen gemäss Anspruch 1, worin $R^1$ Wasserstoff bedeutet.

3. Verbindungen gemäss Anspruch 1 oder 2, worin $R^2$ Wasserstoff bedeutet.

4. Verbindungen gemäss einem der Ansprüche 1-3, worin $R^3$ Wasserstoff bedeutet.

5. Verbindungen gemäss einem der Ansprüche 1-4, worin Y $-CH_2-$ bedeutet.

6. Verbindungen gemäss einem der Ansprüche 1-5, worin $R^1$, $R^2$ und $R^3$ je Wasserstoff und Y $-CH_2-$ bedeuten.

7. Octahydro-9-mercaptomethyl-6,10-dioxo-6H-pyridazo[1,2-a][1,2]diazepin-1-carbonsäure.

8. Octahydro-9-mercaptomethyl-10-oxo-6H-pyridazo[1,2-a][1,2]diazepin--1-carbonsäure.

9. 9-Acetylthiomethyl-octahydro-6,10-dioxo-6H-pyridazo[1,2-a-][1,2]diazepin-1-carbonsäure,

9-Acetylthiomethyl-octahydro-10-oxo-6H-pyridazo[1,2-a][1,-2]diazepin-1-carbonsäure,

tert.Butyl 9-acetylthiomethyl-octahydro-6,10-dioxo-6H-pyridazo[1,2-a]-[1,2]diazepin-1-carboxylat und

tert.Butyl 9-acetylthiomethyl-octahydro-10-oxo-6H-pyridazo[1,2-a][1,-2]diazepin-1-carboxylat.

10. Decahydro-10-mercaptomethyl-6,11-dioxo-6H-pyridazo[1,2-a][1,2]diazocin-1-carbonsäure.

11. tert.Butyl 10-acetylthiomethyl-decahydro-6,11-dioxo-6H-pyridazo[1,2--a][1,2]diazocin-1-carboxylat,

9-Acetylthiomethyl-8-benzyl-octahydro-6,10-dioxo-6H-pyridazo[1,2-a][1,2,5]triazepin-1-carbonsäure,

8-Benzyl-octahydro-9-mercaptomethyl-6,10-dioxo-6H-pyridazo[1,2-a][1,2,5]triazepin-1-carbonsäure,

9-Acetylthiomethyl-8-methyl-octahydro-6,10-dioxo-6H-pyridazo[1,2-a][1,2,5]triazepin-1-carbonsäure,

Octahydro-9-mercaptomethyl-8-methyl-6,10-dioxo-6H-pyridazo[1,2-a][1,2,5]triazepin-1-carbonsäure,

tert.Butyl 9-acetylthiomethyl-8-benzyl-octahydro-6,10-dioxo-6H-pyridazo[1,2-a],1,2,5]triazepin-1-carboxylat,

tert.Butyl 9-acetylthiomethyl-8-methyl-octahydro-6,10-dioxo-6H-pyridazo[1,2-a][1,2,5]triazepin-1-carboxylat und

Methyl 9-acetylthiomethyl-octahydro-6,10-dioxo-6H-pyridazo[1,2-a]-[1,2]diazepin-1-carboxylat.

12. Verbindungen gemäss einem der Ansprüche 1-11, worin die Konfiguration an den asymmetrischen Kohlenstoffatomen, welche die Substituenten $R^1$-S-$CH_2$- und $R^2$ -OOC- tragen, die S-Konfiguration ist.

13. Verbindungen der allgemeinen Formel

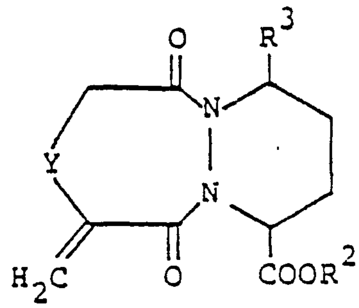

worin $R^2$ Wasserstoff oder Alkyl, $R^3$ Wasserstoff oder Aryl und Y -$CH_2$-, -$CH_2CH_2$- oder -N($R^6$)- bedeuten, worin $R^6$ Wasserstoff, Alkyl oder Aralkyl bedeutet.

14. Verbindungen der allgemeinen Formel

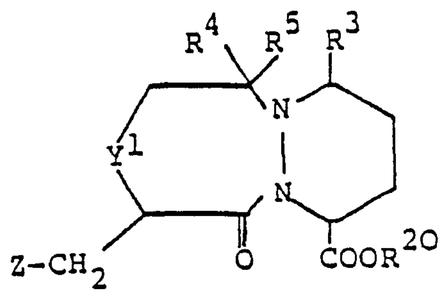

worin $R^3$ Wasserstoff oder Aryl, $R^4$ und $R^5$ je Wasserstoff oder zusammen eine Oxogruppe, $R^{20}$ Alkyl, $Y^1$ -$CH_2$-, -$CH_2CH_2$- oder -N($R^{60}$)-, worin $R^{60}$ Alkyl oder Aralkyl bedeutet, und Z eine Abgangsgruppe bedeuten.

15. Verbindungen der allgemeinen Formel

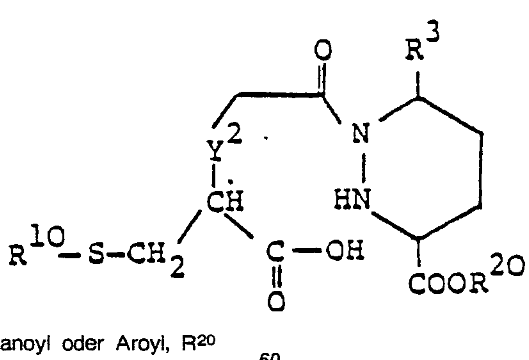

worin $R^3$ Wasserstoff oder Aryl, Alkanoyl oder Aroyl, $R^{20}$ Alkyl und $Y^2$ -$CH_2$- oder -$CH_2CH_2$-bedeuten.

16. Verbindungen der allgemeinen Formel

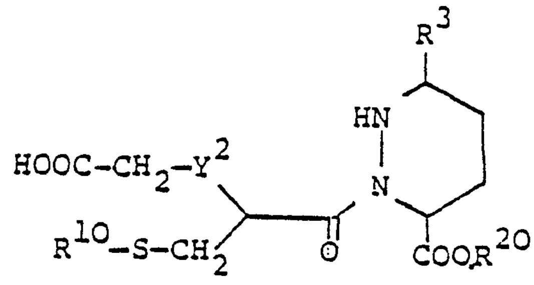

worin $R^3$ Wasserstoff oder Aryl, $R^{10}$ Alkanoyl oder Aroyl, $R^{20}$ Alkyl und $Y^2$ $-CH_2-$ oder $-CH_2CH_2-$bedeuten.

17. Verbindungen der allgemeinen Formel

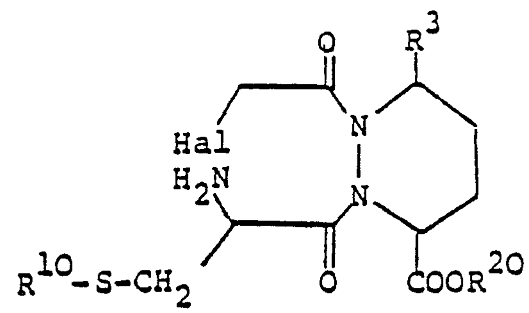

worin $R^3$ Wasserstoff oder Aryl, $R^{10}$ Alkanoyl oder Aroyl, $R^{20}$Alkyl und Hal Halogen bedeuten.

18. Verbindung gemäss einem der Ansprüche 1-12 zur Anwendung als therapeutischer Wirkstoff.

19. Verbindung gemäss einem der Ansprüche 1-12 zur Anwendung als Antihypertensivum.

20. Verfahren zur Herstellung einer Verbindung gemäss einem der Ansprüche 1-12, dadurch gekennzeichnet, dass man

a) zur Herstellung einer Verbindung der Formel I, worin $R^4$ und $R^5$ zusammen eine Oxogruppe bedeuten, eine Verbindung der allgemeinen Formel

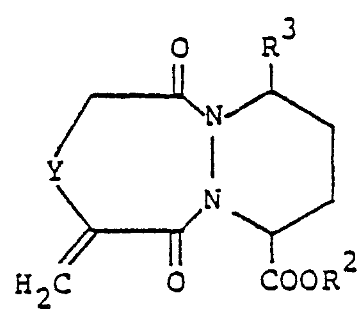

worin $R^2$, $R^3$ und Y die oben angegebene Bedeutung besitzen, mit einer Verbindung der allgemeinen Formel

$R^1-SH$

III

worin $R^1$ die oben angegebene Bedeutung besitzt, umsetzt,

oder

b) zur Herstellung einer Verbindung der Formel I, worin $R^1$ Alkanoyl oder Aroyl, $R^2$ Alkyl und Y $-CH_2-$, $-CH_2CH_2-$ oder $-N(R^6)-$ bedeuten, worin $R^6$ Alkyl oder Aralkyl bedeutet, eine Verbindung der allgemeinen Formel

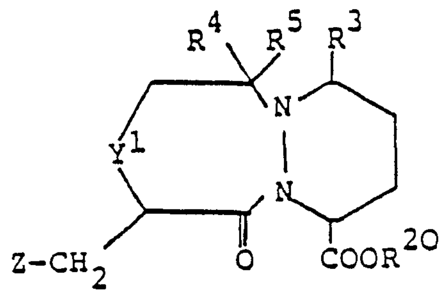

worin $R^3$, $R^4$ und $R^5$ die oben angegebene Bedeutung besitzen, $R^{20}$ Alkyl, $Y^1$ $-CH_2-$, $-CH_2CH_2-$ oder $-N(R^{60})-$, worin $R^{60}$ Alkyl oder Aralkyl bedeutet, und Z eine Abgangsgruppe bedeuten, mit einem Alkalimetallsalz einer Verbindung der allgemeinen Formel

$R^{10}$-SH

V

worin $R^{10}$ Alkanoyl oder Aroyl bedeutet, umsetzt, oder

c) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

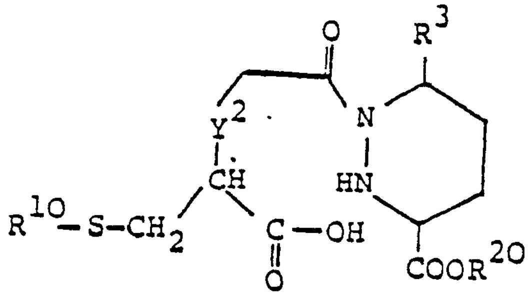

worin $R^3$, $R^{10}$ und $R^{20}$ die oben angegebene Bedeutung besitzen und $Y^2$ $-CH_2-$ oder $-CH_2CH_2-$bedeutet, cyclisiert, oder

d) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

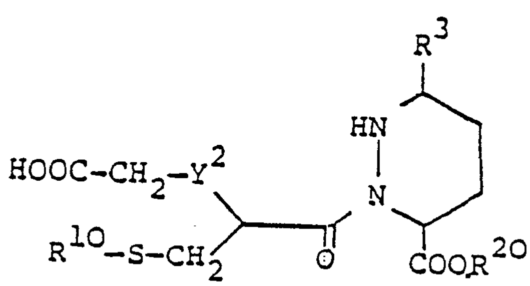

worin $R^3$, $R^{10}$, $R^{20}$ und $Y^2$ die oben angegebene Bedeutung besitzen, cyclisiert, oder

e) zur Herstellung einer Verbindung der Formel I, worin $R^1$ Alkanoyl oder Aroyl, $R^2$ Alkyl, $R^4$ und $R^5$ zusammen eine Oxogruppe und Y -NH- bedeuten, eine Verbindung der allgemeinen Formel

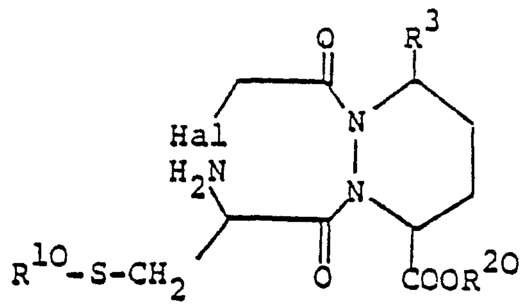

worin $R^3$, $R^{10}$ und $R^{20}$ die oben angegebene Bedeutung besitzen und Hal Halogen bedeutet, cyclisiert, oder

f)zur Herstellung einer Verbindung der Formel I, worin $R^1$ Alkanoyl oder Aroyl, $R^2$ Alkyl und $R^4$ und $R^5$ je Wasserstoff bedeuten, eine Verbindung der Formel I, worin $R^1$ Alkanoyl oder Aroyl, $R^2$ Alkyl und $R^4$ und $R^5$ zusammen eine Oxogruppe bedeuten, reduziert, oder

g) zur Herstellung einer Verbindung der Formel I, worin $R^1$ Alkanoyl oder Aroyl und Y -N($R^6$)- bedeuten, worin $R^6$ Alkyl oder Aralkyl bedeutet, eine Verbindung der Formel I, worin $R^1$ Alkanoyl oder Aroyl und Y -NH- bedeuten, mit einer Verbindung der allgemeinen Formel

$R^{60}$-Z

IX

worin $R^{60}$ und Z die oben angegebene Bedeutung besitzen, umsetzt, oder

h) zur Herstellung einer Verbindung der Formel I, worin $R^1$ Wasserstoff bedeutet, eine Verbindung der Formel I, worin $R^1$ Alkanoyl oder Aroyl bedeutet, mit einer Base behandelt, oder

i) zur Herstellung einer Verbindung der Formel I, worin $R^2$ Wasserstoff bedeutet, eine Verbindung der Formel I, worin $R^2$ Alkyl bedeutet, mit einer Säure oder einer Base behandelt, oder

j) zur Herstellung einer Verbindung der Formel I, worin $R^1$ Alkanoyl oder Aroyl und Y $-CH_2-$, $-CH_2CH_2-$ oder -N($R^6$)- bedeuten, worin $R^6$ Alkyl oder Aralkyl bedeutet, eine entsprechende Verbindung der Formel I, worin $R^1$ Wasserstoff bedeutet, alkanoyliert oder aroyliert, oder

k) erwünschtenfalls ein erhaltenes Gemisch von diastereoisomeren Racematen in die entsprechenden diastereoisomeren Racemate oder optisch reinen Diastereoisomeren auftrennt, und/oder

l) erwünschtenfalls ein erhaltenes Racemat in die beiden optischen Antipoden auftrennt, und

m) erwünschtenfalls eine erhaltene Verbindung der Formel I, worin $R^2$ Wasserstoff bedeutet, in ein pharmazeutisch verwendbares Salz mit einer Base überführt oder eine erhaltene Verbindung der Formel I, worin Y -N($R^6$)- bedeutet, in ein pharmazeutisch verwendbares Salz mit einer Säure überführt.

21. Arzneimittel, enthaltend eine Verbindung gemäss einem der Ansprüche 1-12 und ein therapeutisch inertes Excipiens.

22. Antihypertensivum, enthaltend eine Verbindung gemäss einem der Ansprüche 1-12 und ein therapeutisch inertes Excipiens.

23. Verwendung einer Verbindung gemäss einem der Ansprüche 1-12 zur Herstellung eines Antihypertensivums.

Patentansprüche für den Vertragsstaat: AT

1. Verfahren zur Herstellung von Verbindungen der allgemeinen Formel

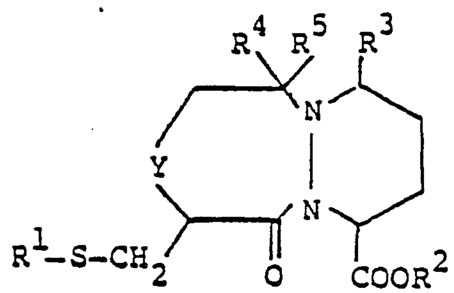

worin $R^1$ Wasserstoff, Alkanoyl oder Aroyl, $R^2$ Wasserstoff oder Alkyl, $R^3$ Wasserstoff oder Aryl, $R^4$ und $R^5$ je Wasserstoff oder zusammen eine Oxogruppe und Y $-CH_2-$, $-CH_2CH_2-$ oder -N($R^6$)- bedeuten, worin $R^6$ Wasserstoff, Alkyl oder Aralkyl bedeutet, und pharmazeutisch verwendbaren Salzen davon mit Basen, falls $R^2$ Wasserstoff bedeutet, und pharmazeutisch verwendbaren Salzen davon mit Säuren, falls Y -N($R^6$)- bedeutet, dadurch gekennzeichnet, dass man

a) zur Herstellung einer Verbindung der Formel I, worin $R^4$ und $R^5$ zusammen eine Oxogruppe bedeuten, eine Verbindung der allgemeinen Formel

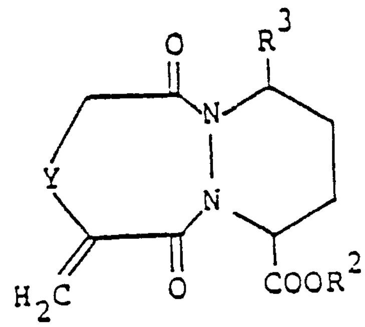

worin $R^2$, $R^3$ und Y die oben angegebene Bedeutung besitzen, mit einer Verbindung der allgemeinen Formel

$R^1$-SH

III

worin $R^1$ die oben angegebene Bedeutung besitzt, umsetzt, oder

b) zur Herstellung einer Verbindung der Formel I, worin $R^1$ Alkanoyl oder Aroyl, $R^2$ Alkyl und Y $-CH_2-$, $-CH_2CH_2-$ oder $-N(R^6)-$ bedeuten, worin $R^6$ Alkyl oder Aralkyl bedeutet, eine Verbindung der allgemeinen Formel

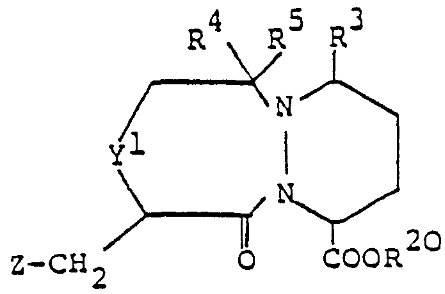

worin $R^3$, $R^4$ und $R^5$ die oben angegebene Bedeutung besitzen, $R^{20}$ Alkyl, $Y^1$ $-CH_2-$, $-CH_2CH_2-$ oder $-N(R^{60})-$, worin $R^{60}$ Alkyl oder Aralkyl bedeutet, und Z eine Abgangsgruppe bedeuten, mit einem Alkalimetallsalz einer Verbindung der allgemeinen Formel

$R^{10}$-SH

V

worin $R^{10}$ Alkanoyl oder Aroyl bedeutet, umsetzt, oder

c) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

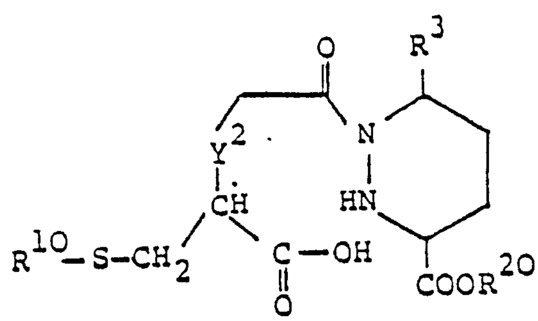

worin $R^3$, $R^{10}$ und $R^{20}$ die oben angegebene Bedeutung besitzen und $Y^2$ $-CH_2-$ oder $-CH_2CH_2-$ bedeutet, cyclisiert, oder

d) zur Herstellung einer Verbindung der Formel I, worin $R^1$ Alkanoyl oder Aroyl, $R^2$ Alkyl, $R^4$ und $R^5$ zusammen eine Oxogruppe und Y $-CH_2-$ oder $-CH_2CH_2-$ bedeuten, eine Verbindung der allgemeinen Formel

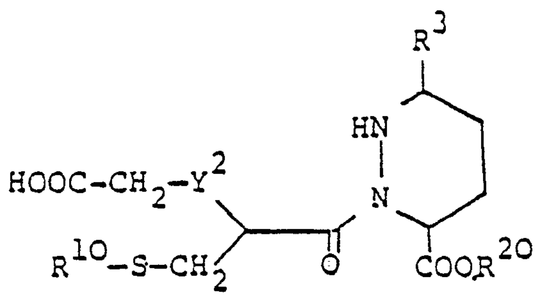

worin $R^3$, $R^{10}$, $R^{20}$ und $Y^2$ die oben angegebene Bedeutung besitzen, cyclisiert, oder

e) zur Herstellung einer Verbindung der Formel I, worin $R^1$ Alkanoyl oder Aroyl, $R^2$ Alkyl, $R^4$ und $R^5$ zusammen eine Oxogruppe und Y -NH- bedeuten, eine Verbindung der allgemeinen Formel

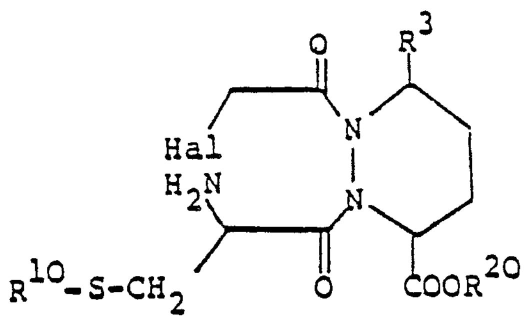

worin $R^3$, $R^{10}$ und $R^{20}$ die oben angegebene Bedeutung besitzen und Hal Halogen bedeutet, cyclisiert, oder

f) zur Herstellung einer Verbindung der Formel I, worin $R^1$ Alkanoyl oder Aroyl, $R^2$ Alkyl und $R^4$ und $R^5$ je Wasserstoff bedeuten, eine Verbindung der Formel I, worin $R^1$ Alkanoyl oder Aroyl, $R^2$ Alkyl und $R^4$ und $R^5$ zusammen eine Oxogruppe bedeuten, reduziert, oder

g) zur Herstellung einer Verbindung der Formel I, worin $R^1$ Alkanoyl oder Aroyl und Y -N($R^6$)- bedeuten, worin $R^6$ Alkyl oder Aralkyl bedeutet, eine Verbindung der Formel I, worin $R^1$ Alkanoyl oder Aroyl und Y -NH- bedeuten, mit einer Verbindung der allgemeinen Formel

$R^{60}$-Z IX

worin $R^{60}$ und Z die oben angegebene Bedeutung besitzen, umsetzt, oder

h) zur Herstellung einer Verbindung der Formel I, worin $R^1$ Wasserstoff bedeutet, eine Verbindung der Formel I, worin $R^1$ Alkanoyl oder Aroyl bedeutet, mit einer Base behandelt, oder

i) zur Herstellung einer Verbindung der Formel I, worin $R^2$ Wasserstoff bedeutet, eine Verbindung der Formel I, worin $R^2$ Alkyl bedeutet, mit einer Säure oder einer Base behandelt, oder

j) zur Herstellung einer Verbindung der Formel I, worin $R^1$ Alkanoyl oder Aroyl und Y $-CH_2-$, $-CH_2CH_2-$ oder -N($R^6$)- bedeuten, worin $R^6$ Alkyl oder Aralkyl bedeutet, eine entsprechende Verbindung der Formel I, worin $R^1$ Wasserstoff bedeutet, alkanoyliert oder aroyliert, oder

k) erwünschtenfalls ein erhaltenes Gemisch von diastereoisomeren Racematen in die entsprechenden diastereoisomeren Racemate oder optisch reinen Diastereoisomeren auftrennt, und/oder

l) erwünschtenfalls ein erhaltenes Racemat in die beiden optischen Antipoden auftrennt, und

m) erwünschtenfalls eine erhaltene Verbindung der Formel I, worin $R^2$ Wasserstoff bedeutet, in ein pharmazeutisch verwendbares Salz mit einer Base überführt oder eine erhaltene Verbindung der Formel I, worin Y -N($R^6$)- bedeutet, in ein pharmazeutisch verwendbares Salz mit einer Säure überführt.

2. Verfahren nach Anspruch 1, worin $R^1$ Wasserstoff bedeutet.

3. Verfahren nach Anspruch 1 oder 2, worin $R^2$ Wasserstoff bedeutet.

4. Verfahren nach einem der Ansprüche 1-3, worin $R^3$ Wasserstoff bedeutet.

5. Verfahren nach einem der Ansprüche 1-4, worin Y $-CH_2-$ bedeutet.

6. Verfahren nach einem der Ansprüche 1-5, worin $R^1$, $R^2$ und $R^3$ je Wasserstoff und Y $-CH_2-$ bedeuten.

7. Verfahren nach Anspruch 1, dadurch gekennzeichnet, dass man Octahydro-9-mercaptomethyl-6,10-dioxo-6H-pyridazo[1,2-a][1,2]diazepin-1-carbonsäure herstellt

8. Verfahren nach Anspruch 1, dadurch gekennzeichnet, dass man Octahydro-9-mercaptomethyl-10-oxo-6H -

pyridazo[1,2-a][1,2]diazepin-1-carbonsäure herstellt.

9. Verfahren nach Anspruch 1, dadurch gekennzeichnet, dass man Decahydro-10-mercaptomethyl-6,11-dioxo-6H -pyridazo[1,2-a][1,2]diazocin-1-carbonsäure herstellt.

10. Verfahren nach einem der Ansprüche 1-9, dadurch gekennzeichnet, dass die Konfiguration an den asymmetrischen Kohlenstoffatomen, welche die Substituenten $R^1$-S-$CH_2$ und $R^2$-OOC tragen. die S-Konfiguration ist.